# EUROPÄISCHE PATENTSCHRIFT

(11) **EP 1 660 509 B1**
(45) Veröffentlichungstag und Bekanntmachung des Hinweises auf die Patenterteilung: **04.02.2009**
(21) Anmeldenummer: 04764316.8
(22) Anmeldetag: 20.08.2004
(51) Int. Cl.: C07H 17/02, A61K 31/7056, A61P 3/10

(54) **GLUCOPYRANOSYLOXY-PYRAZOLE, DIESE VERBINDUNGEN ENTHALTENDE ARZNEIMITTEL, DEREN VERWENDUNG UND VERFAHREN ZU IHRER HERSTELLUNG**
GLUCOPYRANOSYLOXY- PYRAZOLES, PHARMACEUTICAL COMPOSITIONS CONTAINING THESE COMPOUNDS, THE USE THEREOF AND PROCESSES FOR THE PREPARATION THEREOF
PYRAZOLES GLUCOPYRANOSYLOXY, MEDICAMENTS CONTENANT CES COMPOSES, LEUR UTILISATION ET LEUR PROCEDE DE PRODUCTION

(30) Priorität: 26.08.2003 DE 10339549; 18.12.2003 DE 10359960
(43) Veröffentlichungstag der Anmeldung: 31.05.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HIMMELSBACH, Frank, 88441 MITTELBIBERACH (DE); EICKELMANN, Peter, 65191 WIESBADEN (DE); THOMAS, Leo, 88400 BIBERACH (DE); BARSOUMIAN, Edward Leon, TOYONAKA, Osaka 560-0005 (JP)
(74) Vertreter: Hammann, Heinz
(86) Internationale Anmeldenummer: PCT/EP2004/009332
(87) Internationale Veröffentlichungsnummer: WO 2005/021566

(56) Entgegenhaltungen:
- WO-A-02/053573
- WO-A-02/068439
- WO-A-02/068440
- WO-A-02/088157

## Beschreibung

Gegenstand der vorliegenden Erfindung sind Glucopyranosyloxy-pyrazole der allgemeinen Formel I 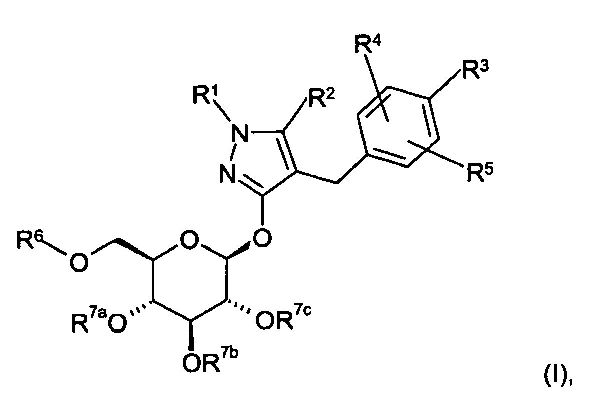 wobei die Reste R¹ bis R⁶ und R^{7a}, R^{7b}, R^{7c} nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Stoffwechselerkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf den natriumabhängigen Glucose-Cotransporter SGLT2 besitzen, zur Behandlung von Krankheiten, insbesondere von Diabetes vorgeschlagen.

Aus den internationalen Offenlegungsschriften WO 02/36602, WO 02/088157, WO 01/16147, WO 02/053573, WO 02/068439, WO 02/068440 sowie WO 02/098893 sind Glucopyranosyloxy-pyrazol-Derivate sowie deren Herstellung und deren mögliche Aktivität als SGLT2-Inhibitoren bekannt.

### Aufgabe der Erfindung

Der vorliegenden Erfindung liegt die Aufgabe zugrunde, neue Glucopyranosyloxy-pyrazol-Derivate aufzuzeigen, insbesondere solche, die eine Aktivität bezüglich des natriumabhängigen Glucose-Cotransporters SGLT, insbesondere SGLT2 besitzen. Eine weitere Aufgabe der vorliegenden Erfindung besteht im Aufzeigen von Glucopyranosyloxy-pyrazol-Derivaten, die *in vitro* und/oder *in vivo* im Vergleich mit bekannten, strukturähnlichen Glucopyranosyloxy-pyrazolen eine erhöhte Hemmwirkung bezüglich des natriumabhängigen Glucose-Cotransporters SGLT2 besitzen und/oder verbesserte pharmakologische Eigenschaften aufweisen.

Ferner ist es eine Aufgabe der vorliegenden Erfindung, neue Arzneimittel bereit zu stellen, welche zur Prophylaxe und/oder Behandlung von Stoffwechselerkrankungen, insbesondere von Diabetes geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind Glucopyranosyloxy-pyrazole der allgemeinen Formel I 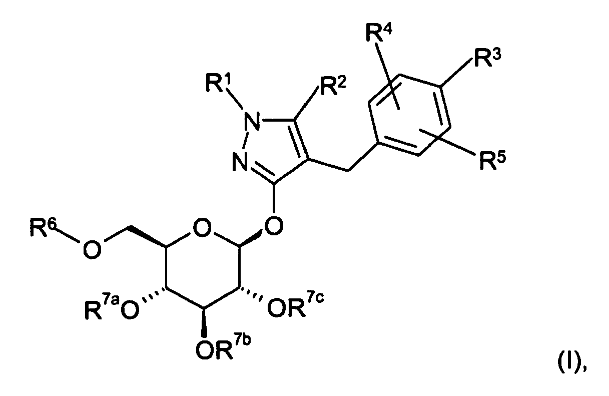 in denen
- R¹: Tetrahydrofuran-3-yl, Tetrahydropyran-3-yl, Tetrahydropyran-4-yl, Tetrahydrofuranyl-C₁₋₃-alkyl-, Tetrahydropyranyl-C₁₋₃-alkyl-, Pyrrolidin-3-yl-, Piperidin-3-yl-, Piperidin-4-yl-, oder
eine Pyrrolidin-3-yl-, Piperidin-3-yl- oder Piperidin-4-yl-Gruppe bedeutet, wobei in den drei zuletzt genannten Gruppen das Stickstoffatom mit einer C₁₋₄-Alkyl-, Formyl-, C₁₋₄-Alkyl-carbonyl-, C₁₋₄-Alkylsulfonyl-, Cyan-, Aminocarbonyl-, (C₁₋₄-Alkyl)-aminocarbonyl-, Di-(C₁₋₄-alkyl)-aminocarbonyl- oder (C₁₋₄-Alkyl)-oxycarbonyl-Gruppe substituiert sein kann, und
- R²: C₁₋₄-Alkyl, eine durch 1 bis 3 Fluoratome substituierte C₁₋₄-Alkyl-Gruppe, oder C₃₋₆-Cycloalkyl bedeutet, und
- R³: Wasserstoff, Fluor, Chlor, Brom, Iod, C₁₋₆-Alkyl, C₂₋₆-Alkenyl, C₂₋₆-Alkinyl, C₃₋₆-Cycloalkyl, C₃₋₆-Cycloalkylidenmethyl, C₁₋₆-Alkoxy, C₃₋₆-Cycloalkyl-oxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, eine durch 1 bis 3 Fluoratome substituierte Methyl- oder Methoxy-Gruppe, eine durch 1 bis 5 Fluoratome substituierte C₂₋₄-Alkyl- oder C₂₋₄-Alkoxy-Gruppe, eine durch eine Cyangruppe substituierte C₁₋₄-Alkyl-Gruppe, eine durch eine Hydroxy- oder C₁₋₃-Alkyloxygruppe substituierte C₁₋₄-Alkyl-Gruppe, Cyan-, Carboxy-, C₁₋₃-Alkoxycarbonyl-, Aminocarbonyl-, (C₁₋₃-Alkylamino)carbonyl-, Di-(C₁₋₃-alkyl)aminocarbonyl-, Pyrrolidin-1-ylcarbonyl-, Piperidin-1-ylcarbonyl-, Morpholin-4-ylcarbonyl-, Piperazin-1-yl-carbonyl-, 4-(C₁₋₃-Alkyl)-piperazin-1-ylcarbonyl-, Nitro-, Amino-, C₁₋₃-Alkylamino-, Di-(C₁₋₃-alkyl)amino-, (C₁₋₄-Alkyl)carbonylamino-, C₁₋₄-Alkylsulfonylamino, Arylsulfonylamino, Aryl-C₁₋₃-alkylsulfonylamino, C₁₋₄-Alkylsulfanyl-, C₁₋₄-Alkylsulfinyl-, C₁₋₄-Alkylsulfonyl, Arylsulfenyl-, Arylsulfinyl- oder Arylsulfonyl-bedeutet, oder
eine Bedeutung ausgewählt aus der Gruppe A besitzt, und
- R⁴ und R⁵,: die gleich oder verschieden sein können, Wasserstoff, Fluor, Chlor, Brom, C₁₋₃-Alkyl, C₁₋₃-Alkoxy, eine durch 1 bis 3 Fluoratome substituierte Methyl- oder Methoxy-Gruppe bedeuten, oder
- R³ zusammen mit R⁴,: sofern diese an benachbarte Kohlenstoffatome gebunden sind, auch eine geradkettige C₃₋₅-Alkylen-, eine Methylendioxy- oder Difluormethylendioxy-Brücke bedeuten können, und
- R⁶, R^{7a}, R^{7b}, R^{7c}: unabhängig voneinander eine Bedeutung ausgewählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl besitzen,
- A: ausgewählt aus der Gruppe bestehend aus Tetrahydrofuran-3-yloxy-, Tetrahydropyran-3-yloxy-, Tetrahydropyran-4-yloxy-, Tetrahydrofuranyl-C₁₋₃-alkoxy-, Tetrahydropyranyl-C₁₋₃-alkoxy-, Pyrrolidin-3-yloxy-, Piperidin-3-yloxy-, Piperidin-4-yloxy-Gruppe, sowie
Pyrrolidin-3-yloxy-, Piperidin-3-yloxy- und Piperidin-4-yloxy-, wobei in den drei zuletzt genannten Gruppen das Stickstoffatom mit C₁₋₄-Alkyl-, Formyl-, C₁₋₄-Alkyl-carbonyl-, C₁₋₄-Alkylsulfonyl-, Cyan-, Aminocarbonyl-, (C₁₋₄-Alkyl)-aminocarbonyl-, Di-(C₁₋₄-alkyl)-aminocarbonyl- oder (C₁₋₄-Alkyl)-oxycarbonyl-substituiert sein kann,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor, Chlor, Brom, Iod, C₁₋₃-Alkyl, Difluormethyl, Trifluormethyl, C₁₋₃-Alkoxy, Difluormethoxy, Trifluormethoxy oder Cyan bedeutet,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen durch Rₕ mono- oder disubstituiert sein können, wobei die Substituenten gleich oder verschieden sein können und Rₕ wie vorstehend definiert ist,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

Die erfindungsgemäßen Verbindungen der allgemeinen Formel I und ihre physiologisch verträglichen Salze weisen wertvolle pharmakologische Eigenschaften auf, insbesondere eine Hemmwirkung auf den natriumabhängigen Glucose-Cotransporter SGLT, insbesondere SGLT2. Ferner können erfindunsgemäße Verbindungen eine Hemmwirkung auf den natriumabhängigen Glucose-Cotransporter SGLT1 aufweisen. Verglichen mit einer möglichen Hemmwirkung auf SGLT1 hemmen die erfindungsgemäßen Verbindungen vorzugsweise selektiv SGLT2.

Gegenstand der vorliegenden Erfindung sind auch die physiologisch verträglichen Salze der erfindungsgemäßen Verbindungen mit anorganischen oder organischen Säuren.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des natriumabhängigen Glucose-Cotransporters SGLT, insbesondere SGLT2 beeinflussbar sind.

Ein weiterer Gegenstand dieser Erfindung ist die Verwendung mindestens einer erfindungsgemäßen Verbindung zur Herstellung eines Arzneimittels, das zur Behandlung von Stoffwechselerkrankungen geeignet ist.

Ein weiterer Gegenstand dieser Erfindung ist die Verwendung mindestens einer erfindungsgemäßen Verbindung zur Herstellung eines Arzneimittels zur Inhibition des natriumabhängigen Glucose-Cotransporters SGLT, insbesondere SGLT2.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, dadurch gekennzeichnet, dass
a) zur Herstellung von Verbindungen der allgemeinen Formel I, in der R¹ wie zuvor definiert ist,
   eine Verbindung der allgemeinen Formel 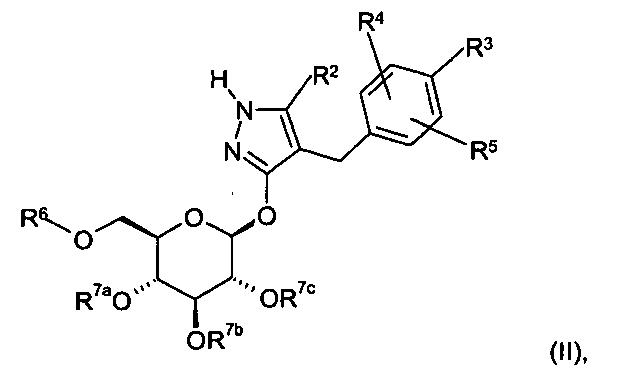 in der
   R² bis R⁶ sowie R^{7a}, R^{7b}, R^{7c} wie zuvor definiert sind, mit einer Verbindung der allgemeinen Formel

      Z¹-R^{1'} (III),

      in der
   R^{1'} die eingangs für R¹ erwähnten Bedeutungen besitzt, und Z¹ eine Austrittsgruppe darstellt, umgesetzt wird oder
b) zur Herstellung von Verbindungen der allgemeinen Formel I, in der R³ gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die wie vorstehend erwähnt definiert ist, darstellt,
   eine Verbindung der allgemeinen Formel 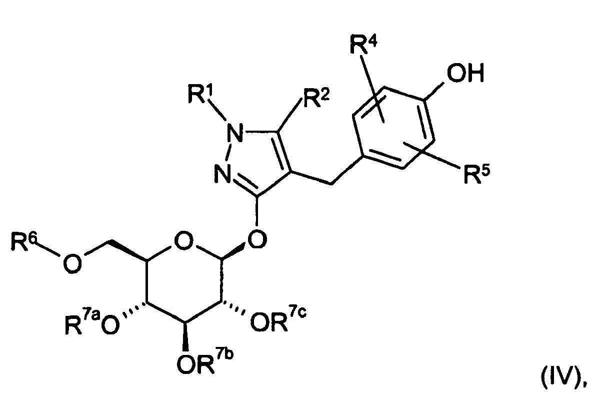 in der
   R¹, R² und R⁴ bis R⁶ sowie R^{7a}, R^{7b}, R^{7c} wie eingangs erwähnt definiert sind, mit einer Verbindung der allgemeinen Formel

      Z² - R^{3'} (V),

      in der
   R^{3'} gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die wie vorstehend erwähnt definiert ist, darstellt, und Z² eine Austrittsgruppe, vorzugsweise ein Halogenatom, beispielsweise ein Chlor- oder Bromatom, eine Sulfonyloxygruppe, beispielsweise eine Methansulfonyloxy- oder p-Toluolsulfonyloxygruppe oder eine Hydroxygruppe darstellt, umgesetzt wird und
nach Durchführung des Schrittes a) oder b) gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
erforderlichenfalls ein bei den vorstehend beschriebenen Umsetzungen verwendeter Schutzrest wieder abgespalten wird und/oder gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, überführt wird.

### Detailierte Beschreibung der Erfindung

Sofern nicht anders angegeben besitzen die Gruppen, Reste und Substituenten, insbesondere R¹ bis R⁶ sowie R^{7a}, R^{7b}, R^{7c}, die zuvor und nachfolgend angegebenen Bedeutungen.

Kommen Reste, Substituenten oder Gruppen in einer Verbindung mehrfach vor, so können diese eine gleiche oder verschiedene Bedeutungen aufweisen.

Die vorstehend und nachfolgend verwendete, beispielsweise in den Gruppen R¹, R³, R⁶, R^{7a} , R^{7b} , R^{7c} vorkommende Bezeichnung Aryl bedeutet vorzugsweise Phenyl. Gemäß der allgemeinen Definition und sofern nichts anderes angegeben ist, kann die Aryl-Gruppe, insbesondere die Phenylgruppe, ein- oder zweifach mit gleichen oder verschiedenen Resten Rₕ substituiert sein.

Erfindungsgemäße Verbindungen können beschrieben werden durch die allgemeine Formel I, in der
- R¹: Tetrahydrofuran-3-yl, Tetrahydropyran-3-yl, Tetrahydropyran-4-yl, Tetrahydrofuranyl-C₁₋₃-alkyl-, Tetrahydropyranyl-C₁₋₃-alkyl-, Pyrrolidin-3-yl-, Piperidin-3-yl-, Piperidin-4-yl-, oder
eine Pyrrolidin-3-yl-, Piperidin-3-yl- oder Piperidin-4-yl-Gruppe bedeutet, wobei in den drei zuletzt genannten Gruppen das Stickstoffatom mit einer C₁₋₄-Alkyl-, Formyl-, C₁₋₄-Alkyl-carbonyl-, C₁₋₄-Alkylsulfonyl-, Cyan-, Aminocarbonyl-, (C₁₋₄-Alkyl)-aminocarbonyl-, Di-(C₁₋₄-alkyl)-aminocarbonyl- oder (C₁₋₄-Alkyl)-oxycarbonyl-Gruppe substituiert sein kann, und
die übrigen Reste R² bis R⁶ sowie R^{7a}, R^{7b}, R^{7c} wie zuvor definiert sind,
einschließlich deren Tautomere, deren Stereoisomere, deren Gemische und deren Salze.

Bevorzugte Bedeutungen des Rests R¹ sind Tetrahydrofuran-3-yl, Tetrahydropyran-3-yl, Tetrahydropyran-4-yl, Tetrahydrofuranylmethyl und Tetrahydropyranylmethyl. Ganz besonders bevorzugte Bedeutungen sind hierbei Tetrahydrofuran-3-yl-, Tetrahydropyran-4-yl- und Tetrahydrofuran-2-ylmethyl-.

Bevorzugte Bedeutungen des Rests R³ sind Wasserstoff, Fluor, Chlor, Methyl, Ethyl, Isopropyl, tert.-Butyl, 2-Cyan-2-propyl, Difluormethyl, Trifluormethyl, Cyclopropyl, Cyclobutyl, Cyclopentyl, Methoxy, Ethoxy, Isopropoxy, Difluormethoxy, Trifluormethoxy, 1,1,2,2-Tetrafluorethoxy, Cyclopropyl-oxy, Cyclobutyl-oxy, Cyclopentyl-oxy, Tetrahydrofuran-3-yloxy, Tetrahydropyran-3-yloxy, Tetrahydropyran-4-yloxy, Tetrahydrofuranylmethyloxy, Tetrahydropyranyl-methyloxy, Methylsulfanyl, 2-Methyl-1-propen-1-yl, Cyclopropylidenmethyl-, Ethinyl, Phenyl-, Fluorphenyl, Pyridyl und Methylthiazolyl. Ganz besonders bevorzugte Bedeutungen sind hierbei Fluor, Chlor, Methyl, Ethyl, Isopropyl, tert.-Butyl, Difluormethyl, Trifluormethyl, Cyclopropyl, Cyclobutyl, Cyclopentyl, Methoxy, Ethoxy, Isopropoxy, Difluormethoxy, Trifluormethoxy, insbesondere Ethyl.

Bevorzugte Bedeutungen des Rests R⁴ sind Wasserstoff und Fluor, insbesondere Wasserstoff.

Besitzt die Gruppe R¹ die Bedeutung Pyrrolidin-3-yl, Piperidin-3-yl oder Piperidin-4-yl, so ist das Stickstoffatom vorzugsweise wie angegeben substituiert.

Gemäß einer Variante sind diejenigen Verbindungen auch bevorzugt, in denen die Phenylgruppe, die den Substituenten R³ trägt, mindestens einen weiteren, von Wasserstoff verschiedenen Substituenten R⁴ und/oder R⁵ aufweist. Nach dieser Variante sind diejenigen Verbindungen besonders bevorzugt, die einen Substituenten R⁴ in der Bedeutung Fluor aufweisen.

Bevorzugte Bedeutungen des Rests R⁵ sind Wasserstoff und Fluor.

Erfindungsgemäß bevorzugte Bedeutungen des Rests R² sind Methyl und Trifluormethyl, insbesondere Methyl.

Der Rest R⁶ bedeutet erfindungsgemäß vorzugsweise Wasserstoff, (C₁₋₈-Alkyl)oxycarbonyl- oder C₁₋₈-Alkylcarbonyl-, insbesondere H oder (C₁₋₆-Alkyl)oxycarbonyl, besonders bevorzugt H, Methoxycarbonyl oder Ethoxycarbonyl.

Die Substituenten R^{7a}, R^{7b}, R^{7c} bedeuten unabhängig voneinander vorzugsweise Wasserstoff oder (C₁₋₁₈-Alkyl)carbonyl, insbesondere Wasserstoff oder (C₁₋₈-Alkyl)carbonyl, besonders bevorzugt Wasserstoff, Methylcarbonyl oder Ethylcarbonyl. Ganz besonders bevorzugt bedeuten R^{7a}, R^{7b} und R^{7c} Wasserstoff.

Die Verbindungen der Formel I, in denen R⁶, R^{7a}, R^{7b} und R^{7c} eine erfindungsgemäße, von H verschiedene Bedeutung aufweisen, beispielsweise C₁₋₈-Alkylcarbonyl, eignen sich bevorzugt als Zwischenprodukte bei der Synthese von Verbindungen der Formel I in denen R^{7a}, R^{7b} und R^{7c} Wasserstoff bedeuten.

Erfindungsgemäß sind Verbindungen der allgemeinen Formel I bevorzugt, in denen
- R¹: Tetrahydrofuran-3-yl, Tetrahydropyran-3-yl, Tetrahydropyran-4-yl, Tetrahydrofuranylmethyl oder Tetrahydropyranylmethyl bedeutet,
- R²: Methyl- oder Trifluormethyl bedeutet,
- R³: Wasserstoff, Fluor, Chlor, Methyl, Ethyl, Isopropyl, tert.-Butyl, 2-Cyan-2-propyl, Difluormethyl, Trifluormethyl, Cyclopropyl, Cyclobutyl, Cyclopentyl, Methoxy, Ethoxy, Isopropoxy, Difluormethoxy, Trifluormethoxy, 1,1,2,2-Tetrafluorethoxy, Cylopropyl-oxy, Cyclobutyl-oxy, Cyclopentyl-oxy, Tetrahydrofuran-3-yloxy, Tetrahydropyran-3-yloxy, Tetrahydropyran-4-yloxy, Tetrahydrofuranylmethyloxy, Tetrahydropyranylmethyloxy, Methylsulfanyl, 2-Methyl-1-propen-1-yl, Cyclopropylidenmethyl-, Ethinyl, Phenyl-, Fluorphenyl, Pyridyl oder Methylthiazolyl bedeutet, und
- R⁴: Wasserstoff- oder Fluor bedeutet, oder,
- R³ zusammen mit R⁴,: sofern diese an benachbarte Kohlenstoffatome gebunden sind, auch eine 1,3-Propylen-, Methylendioxy- oder Difluormethylendioxy-Brücke bedeuten kann, und
- R⁵: Wasserstoff und
- R⁶: Wasserstoff, (C₁₋₈-Alkyl)oxycarbonyl- oder C₁₋₈-Alkylcarbonyl- bedeutet,
deren Tautomere, deren Stereoisomere, deren Gemische und deren Salze.

Erfindungsgemäß sind Verbindungen der allgemeinen Formel I besonders bevorzugt, in denen
- R¹: Tetrahydrofuran-3-yl-, Tetrahydropyran-4-yl- oder Tetrahydrofuran-2-ylmethyl-
- R²: Methyl,
- R³: Methyl, Ethyl, Methoxy, Ethoxy, Difluormethoxy, Tetrahydrofuran-3-yloxy, Tetrahydropyran-4-yloxy oder Tetrahydrofuranylmethyloxy,
- R⁴: Wasserstoff oder Fluor
- R⁵: Wasserstoff und
- R⁶: Wasserstoff, Methoxycarbonyl oder Ethoxycarbonyl bedeutet,
deren Tautomere, deren Stereoisomere, deren Gemische und deren Salze.

Erfindungsgemäß sind Verbindungen der allgemeinen Formel I ganz besonders bevorzugt, in denen
- R¹: Tetrahydrofuran-3-yl oder Tetrahydropyran-4-yl,
- R²: Methyl,
- R³: Methyl, Ethyl, Methoxy, Ethoxy oder Difluormethoxy,
- R⁴: Wasserstoff oder Fluor,
- R⁵: Wasserstoff,
- R⁶: Wasserstoff, Methoxycarbonyl oder Ethoxycarbonyl und
- R^{7a}, R^{7b} und R^{7c}: Wasserstoff bedeuten,
deren Tautomere, deren Stereoisomere, deren Gemische und deren Salze.

Besonders bevorzugte Verbindungen der allgemeinen Formel I sind ausgewählt aus der Gruppe:
(a) 1-((S)-Tetrahydrofuran-3-yl)-3-(β-D-glucopyranosyloxy)-4-(4-ethylbenzyl)-5-methyl-1 H-pyrazol
(b) 1-(Tetrahydropyran-4-yl)-3-(β-D-glucopyranosyloxy)-4-(4-ethylbenzyl)-5-methyl-1 H-pyrazol,
(c) 1-(Tetrahydropyran-4-yl)-3-(β-D-glucopyranosyloxy)-4-(4-methoxybenzyl)-5-methyl-1 H-pyrazol,
sowie deren Derivate, in denen R⁶ eine erfindungsgemäße, von Wasserstoff verschiedene Bedeutung aufweist, insbesondere R⁶ Ethoxycarbonyl oder Methoxycarbonyl bedeutet,
einschließlich deren Tautomere, deren Stereoisomere, deren Gemische und deren Salze.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und I, insbesondere F, Cl und Br.

Die Bezeichnung C₁₋ₙ-Alkyl, wobei n einen Wert von 2 bis 8 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Die Bezeichnung C₁₋ₙ-Alkylen, wobei n einen Wert von 2 bis 8 besitzen kann, bedeutet eine gesättigte, verzweigte oder unverzweigte Kohlenwasserstoffbrücke mit 1 bis n C-Atomen. Beispiele solcher Gruppen umfassen Methylen (-CH₂-), Ethylen (-CH₂-CH₂-), 1-Methyl-ethylen (-CH(CH₃)-CH₂-), 1,1-Dimethyl-ethylen (-C(CH₃)₂-CH₂-), n-Prop-1,3-ylen (-CH₂-CH₂-CH₂-), 1-Methylprop-1,3-ylen (-CH(CH₃)-CH₂-CH₂-), 2-Methylprop-1,3-ylen (-CH₂-CH(CH₃)-CH₂-), etc., sowie die entsprechenden spiegelbildlichen Formen.

Der Begriff C₂₋ₙ-Alkenyl, wobei n einen Wert von 3 bis 6 besitzt, bezeichnet eine verzweigte oder unverzweigte Kohlenwasserstoffgruppe mit 2 bis n C-Atomen und einer C=C-Doppelbindung. Beispiele solcher Gruppen umfassen Vinyl, 1-Propenyl, 2-Propenyl, iso-Propenyl, 1-Butenyl, 2-Butenyl, 3-Butenyl, 2-Methyl-1-propenyl, 1-Pentenyl, 2-Pentenyl, 3-Pentenyl, 4-Pentenyl, 3-Methyl-2-butenyl, 1-Hexenyl, 2-Hexenyl, 3-Hexenyl, 4-Hexenyl-, 5-Hexenyl etc..

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, iso-Propinyl, 1-Butinyl, 2-Butinyl, 3-Butinyl, 2-Methyl-1-propinyl, 1-Pentinyl, 2-Pentinyl, 3-Pentinyl, 4-Pentinyl, 3-Methyl-2-butinyl, 1-Hexinyl, 2-Hexinyl, 3-Hexinyl, 4-Hexinyl-, 5-Hexinyl etc..

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen.

Der Begriff C₅₋ₙ-Cycloalkenyl bezeichnet eine einfach ungesättigte mono-, bi-, tri- oder spirocarbocyclische Gruppe mit 5 bis n C-Atomen. Beispiele solcher Gruppen umfassen Cyclopentenyl, Cyclohexenyl, Cycloheptenyl, Cyclooctenyl, Cyclononenyl, etc..

Der Begriff C₃₋ₙ-Cycloalkyloxy bezeichnet eine C₃₋ₙ-Cycloalkyl-O-Gruppe, worin C₃₋ₙ-Cycloalkyl wie oben definiert ist. Beispiele solcher Gruppen umfassen Cyclopropyloxy, Cyclobutyloxy, Cyclopentyloxy, Cyclohexyloxy, Cycloheptyloxy, etc..

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist.

Die vorstehend und nachfolgend verwendete Schreibweise, bei der in einer Phenylgruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.
a) Zur Herstellung von Verbindungen der allgemeinen Formel I, in der R¹ wie eingangs erwähnt definiert ist:
   Umsetzung einer Verbindung der allgemeinen Formel 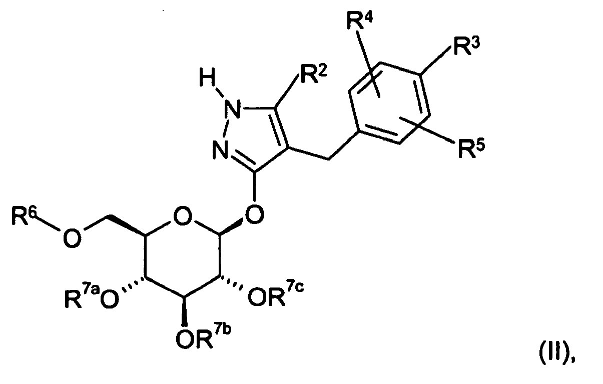 in der
      R² bis R⁶ sowie R^{7a}, R^{7b}, R^{7c} wie eingangs definiert sind, mit einer Verbindung der allgemeinen Formel

         Z¹ - R^{1'} (III),

         in der
      R^{1'} die eingangs für R¹ erwähnten Bedeutungen besitzt, und Z¹ eine Austrittsgruppe, vorzugsweise ein Halogenatom, beispielsweise ein Chlor- oder Bromatom, eine Sulfonyloxygruppe, beispielsweise eine Methansulfonyloxy- oder p-Toluolsulfonyloxygruppe oder eine Hydroxygruppe, darstellt.
   Die Umsetzung erfolgt zweckmäßigerweise in einem Lösungsmittel, wie beispielsweise Acetonitril, Toluol, Tetrahydrofuran, Dioxan, Dimethylformamid, Dimethylsulfoxid oder N-Methylpyrrolidinon, gegebenfalls in Gegenwart einer Base, wie beispielsweise Kaliumcarbonat, Cesiumcarbonat, Natriumhydrid oder Kalium-tert.-butylat, bei Temperaturen zwischen 20°C und 160°C.
   Mit einer Verbindung der allgemeinen Formel III, in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether oder Gemischen aus diesen bei Temperaturen zwischen -50 und 150°C, vorzugsweise jedoch bei Temperaturen zwischen -20 und 80°C, durchgeführt.
b) Zur Herstellung von Verbindungen der allgemeinen Formel I, in der R³ gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die wie vorstehend erwähnt definiert ist, darstellt:
   Umsetzung einer Verbindung der allgemeinen Formel 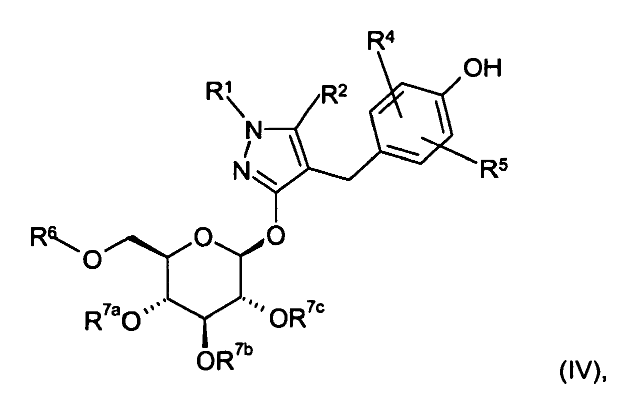 in der
      R¹, R² und R⁴ bis R⁶ sowie R^{7a}, R^{7b}, R^{7c} wie eingangs definiert sind, mit einer Verbindung der allgemeinen Formel

         Z² - R^{3'} (V),

         in der
      R^{3'} gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die wie vorstehend erwähnt definiert ist, darstellt, und Z² eine Austrittsgruppe, vorzugsweise ein Halogenatom, beispielsweise ein Chlor- oder Bromatom, eine Sulfonyloxygruppe, beispielsweise eine Methansulfonyloxy- oder p-Toluolsulfonyloxygruppe oder eine Hydroxygruppe, darstellt.

Die Umsetzung erfolgt zweckmäßigerweise in einem Lösungsmittel, wie beispielsweise Acetonitril, Toluol, Tetrahydrofuran, Dioxan, Dimethylformamid, Dimethylsulfoxid oder N-Methylpyrrolidinon, gegebenfalls in Gegenwart einer Base, wie beispielsweise Kaliumcarbonat, Cesiumcarbonat, Natriumhydrid oder Kalium-tert.-butylat, bei Temperaturen zwischen 20°C und 160°C.
Mit einer Verbindung der allgemeinen Formel V, in der Z² eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether oder Gemischen aus diesen bei Temperaturen zwischen -50 und 150°C, vorzugsweise jedoch bei Temperaturen zwischen -20 und 80°C, durchgeführt.

Erhält man erfindungsgemäß eine Verbindung der allgemeinen Formel I, in der R⁶ ein Wasserstoffatom darstellt, so kann diese mittels Acylierung, beispielsweise mittels Acylierung in Gegenwart einer Base wie Pyridin, Collidin, Triethylamin oder N-Ethyldiisopropylamin, in eine Verbindung übergeführt werden, in der R⁶ eine (C₁₋₁₈-Alkyl)carbonylgruppe, eine (C₁₋₁₈-Alkyl)oxycarbonylgruppe, eine Arylcarbonylgruppe oder eine Aryl-(C₁₋₃-alkyl)-carbonylgruppe darstellt. Als Acylierungsmittel kommen insbesondere die entsprechenden aktivierten Acylderivate wie Säurechloride oder Anhydride in Betracht.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch vorteilhaft hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Weiterhin können die erhaltenen Verbindungen in Gemische, beispielsweise in 1:1 oder 1:2 Gemische mit Aminosäuren, insbesondere mit alpha-Aminosäuren wie Prolin oder Phenylalanin, übergeführt werden, die besonders günstige Eigenschaften wie hohe Kristallinität aufweisen können.

Die als Ausgangsstoffe verwendeten Verbindungen der allgemeinen Formeln II bis V sind teilweise literaturbekannt oder können nach an sich literaturbekannten Verfahren (siehe Beispiele I bis VI), gegebenenfalls unter zusätzlicher Einführung von Schutzresten, erhalten werden.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren, insbesondere den in den WO 02/36602, WO 02/088157, WO 01/16147, WO 02/053573, WO 02/068439, WO 02/068440 sowie WO 02/098893 beschriebenen Verfahren, kombiniert werden können.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf den natriumabhängigen Glucose-Cotransporter SGLT, vorzugsweise SGLT2.

Die biologischen Eigenschaften der neuen Verbindungen können wie folgt geprüft werden:

Die Fähigkeit der Substanzen die SGLT-2 Aktivität zu hemmen, kann in einem Versuchsaufbau gezeigt werden, in dem eine CHO-K1 Zelllinie (ATCC No. CCL 61) oder alternativ eine HEK293 Zelllinie (ATCC No. CRL-1573), die stabil mit einem Expressionsvektor pZeoSV (Invitrogen, EMBL accession number L36849) transfiziert ist, der die cDNA für die kodierende Sequenz des humanen Natrium Glucose Cotransporters 2 (Genbank Acc. No.NM_003041) enthält (CHO-hSGLT2 bzw. HEKhSGLT2). Diese Zelllinien transportieren Natrium-abhängig ¹⁴C-markiertes alpha-Methyl-Glucopyranosid (¹⁴C-AMG, Amersham) in das Zellinnere.

Der SGLT-2 Assay wird wie folgt durchgeführt:
CHO-hSGLT2 Zellen werden in Ham's F12 Medium (BioWhittaker) mit 10% fötalem Kälberserum und 250 µg/ml Zeocin (Invitrogen), HEK293-hSGLT2 Zellen in DMEM Medium mit 10% fötalem Kälberserum und 250 µg/ml Zeocin (Invitrogen) kultiviert. Die Zellen werden von den Kulturflaschen durch zweimaliges Waschen mit PBS und anschließende Behandlung mit Trypsin/EDTA abgelöst. Nachzugabe von Zellkulturmedium werden die Zellen abzentrifugiert, in Kulturmedium resuspendiert und in einem Casy-cell-counter gezählt. Anschließend werden 40.000 Zellen pro Loch in eine weiße, Poly-D-Lysin beschichtete 96-Loch Platte ausgesät und über Nacht bei 37°C, 5% CO₂ inkubiert. Die Zellen werden zweimal mit 250µl Assaypuffer (Hanks Balanced Salt Solution, 137 mM NaCl, 5,4 mM KCI, 2,8 mM CaCl₂, 1,2 mM MgSO₄ und 10 mM HEPES (pH7,4), 50µg/ml Gentamycin) gewaschen. In jedes Loch werden dann 250 µl Assaypuffer und 5 µl Testverbindung hinzugegeben und für weitere 15 Minuten im Brutschrank inkubiert. Als Negativkontrolle werden 5 µl 10% DMSO eingesetzt. Durch Zugabe von 5 µl ¹⁴C-AMG (0.05 µCi) in jedes Loch wird die Reaktion gestartet. Nach einer 2 stündigen Inkubation bei 37°C, 5% CO₂ werden die Zellen wiederum mit 250 µl PBS (20°C) gewaschen und anschließend durch Zugabe von 25 µl 0.1 N NaOH lysiert (5 min. bei 37°C). Pro Loch werden 200 µl MicroScint20 (Packard) hinzugefügt und für weitere 20 min bei 37°C inkubiert. Nach dieser Inkubation wird die Radioaktivität des aufgenommenen ¹⁴C-AMG in einem Topcount (Packard) mittels eines ¹⁴C-Szintillationsprogramms gemessen.

Zur Bestimmung der Selektivität gegenüber dem humanen SGLT1 wird ein analoger Test aufgebaut, in dem die cDNA für hSGLT1 (Genbank Acc. No. NM000343) statt der hSGLT2 cDNA in CHO-K1 bzw. HEK293 Zellen exprimiert wird.

Alternativ kann für hSGLT1 und hSGLT2 auch die Messung des zellulären Membranpotentials zur biologischen Testung von Substanzen herangezogen werden. Hierzu können die weiter oben beschriebenen Zellmodelle angewendet werden. Für den Test werden 10.000 Zellen pro Loch einer poly-D-Lysin beschichteten schwarzen 384-Loch-Platte mit durchsichtigem Boden in Kulturmedium ausgesät und 16 Stunden bei 37°C, 5% CO₂ inkubiert. Anschließend werden die Zellen zweimal mit glucosefreiem HBSS Puffer (12,67 mol/l CaCl₂, 4,93 mmol/l MgCl₂, 4,07 mmol/l MgSO₄, 4,41 mmol/l KH₂PO₄; pH 7,4) gewaschen und mit 20µl HBSS überschichtet. Nach Zugabe von 20 µl Ladepuffer (Membrane Potential Assay Kit Explorer R8126, Molecular Devices GmbH, Ismaning) und 20 µl der zu testenden Substanz in geeigneter Konzentration wird für weitere 30 min. bei 37°C, 5% CO₂ inkubiert. Die Messung erfolgt im Fluorescent Imaging Plate Reader (Molecular Devices GmbH, Ismaning) bei 485 nm Anregungswellenlänge und wird durch Zugabe von 20µl Stimulationspuffer (140 mM NaCl und 120 mM Glucose) gestartet. Die durch den Glucose-induzierten Na⁺-Einstrom verursachte Depolarisation der Zelle kann als Fluoreszenzänderung gemessen und quantifiziert werden.

Die erfindungsgemäßen Verbindungen der allgemeinen Formel I können beispielsweise EC50-Werte unter 1000 nM, insbesondere unter 200 nM, besonders bevorzugt unter 50 nM aufweisen. Beispielsweise weist die Verbindung (4) des Beispiels 3 einen EC50-Wert von etwa 5 nM im SGLT-2-Assay auf.

Im Hinblick auf die Fähigkeit, die SGLT Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der SGLT Aktivität, insbesondere der SGLT-2 Aktivität beeinflusst werden können. Daher sind erfindungsgemäße Verbindungen insbesondere zur Prophylaxe oder Behandlung von Krankheiten, insbesondere Stoffwechselerkrankungen, oder Zuständen wie Diabetes mellitus Typ 1 und Typ 2, diabetische Komplikationen (wie z.B. Retinopathie, Nephropathie oder Neuropathien, diabetischer Fuß, Ulcus, Makroangiopathien), metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet. Darüber hinaus sind diese Substanzen geeignet, die beta-Zelldegeneration wie z.B. Apoptose oder Nekrose von pankreatischen beta-Zellen zu verhindern. Die Substanzen sind weiter geeignet, die Funktionalität von pankreatischen Zellen zu verbessern oder wiederherzustellen, daneben die Anzahl und Größe von pankreatischen beta-Zellen zu erhöhen. Die erfindungsgemäßen Verbindungen sind ebenfalls als Diuretika oder Antihypertensiva einsetzbar und zur Prophylaxe und Behandlung des akuten Nierenversagens geeignet.

Der Patient, dessen Krankheit oder Zustand es erfindungsgemäß zu behandeln oder vorzubeugen gilt, ist ein Säugetier, insbesondere ein Mensch.

Ganz besonders sind die erfindungsgemäßen Verbindungen, einschließlich deren physiologisch verträglichen Salze, zur Prophylaxe oder Behandlung von Diabetes, insbesondere Diabetes mellitus Typ 1 und Typ 2, und/oder diabetischen Komplikationen geeignet.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 1 bis 100 mg, vorzugsweise 1 bis 30 mg, und bei oraler Gabe im Bereich von 1 bis 1000 mg, vorzugsweise 1 bis 100 mg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Lösungen, Suspensionen oder Zäpfchen einarbeiten.

Die erfindungsgemäßen Verbindungen können auch in Kombination mit anderen Wirkstoffen, insbesondere zur Behandlung und/oder Prophylaxe der zuvor angegebenen Krankheiten und Zustände verwendet werden. Für solche Kombinationen kommen als weitere Wirksubstanzen insbesondere solche in Betracht, die beispielsweise die therapeutische Wirksamkeit eines erfindungsgemäßen SGLT-Antagonisten im Hinblick auf eine der genannten Indikationen verstärken und/oder die eine Reduzierung der Dosierung eines erfindungsgemäßen SGLT-Antagonisten erlauben. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose), DPPIV Inhibitoren (z.B. LAF237, MK-431), alpha2-Antagonisten, Insulin und Insulinanaloga, GLP-1 und GLP-1 Analoga (z.B. Exendin-4) oder Amylin. Daneben sind weitere als Kombinationspartner geeignete Wirkstoffe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder β3-Agonisten wie SB-418790 oder AD-9677 ebenso wie Agonisten des 5HT2c Rezeptors.

Daneben ist eine Kombination mit Medikamenten zur Beeinflussung des Bluthochdrucks, des chronischen Herzversagens oder der Atherosklerose wie z.B. A-II Antagonisten oder ACE Inhibitoren, ECE-Inhibitoren, Diuretika, β-Blocker, Ca-Antagonisten, zentral wirksamen Antihypertensiva, Antagonisten des alpha-2-adrenergen Rezeptors, Inhibitoren der neutralen Endopeptidase, Thrombozytenaggregationshemmer und anderen oder Kombinationen daraus geeignet. Beispiele von Angiotensin II Rezeptor Antagonisten sind Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701, etc.. Angiotensin II Rezeptor Antagonisten werden vorzugsweise zur Behandlung oder Prophylaxe von Bluthochdruck und diabetischen Komplikationen verwendet, oft in Kombination mit einem Diuretikum wie Hydrochlorothiazide.

Zur Behandlung oder Prophylaxe der Gicht ist eine Kombination mit Harnsäuresynthese Inhibitoren oder Urikosurika geeignet.

Zur Behandlung oder Prophylaxe diabetischer Komplikationen kann eine Kombination mit GABA-Rezeptor-Antagonisten, Na-Kanal-Blockern, Topiramat, Protein-Kinase C Inhibitoren, advanced glycation endproduct Inhibitoren oder Aldose Reduktase Inhibitoren erfolgen.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind. Hierbei handelt es sich vorzugsweise um eine Stoffwechselerkrankung, insbesondere eine der zuvor angeführten Erkrankungen oder Zustände, ganz besonders Diabetes oder diabetischer Komplikationen.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen und/oder Verdünnungsmitteln aufweist.

So weist beispielsweise ein erfindungsgemäßes Arzneimittel eine Kombination aus einer erfindungsgemäßen Verbindung der Formel I oder eines physiologisch verträglichen Salzes solch einer Verbindung sowie mindestens einem Angiotensin II Rezeptor Antagonisten neben gegebenenfalls einem oder mehreren inerten Trägerstoffen und/oder Verdünnungsmitteln auf.

Die erfindungsgemäße Verbindung, oder eines physiologisch verträglichen Salzes, und der damit zu kombinierende weitere Wirkstoff können zusammen in einer Darreichungsform, beispielsweise einer Tablette oder Kapsel, oder getrennt in zwei gleichen oder verschiedenen Darreichungsformen, beispielsweise als sogenanntes kitof-parts, vorliegen.

Vorstehend und nachfolgend werden in Strukturformeln H-Atome von Hydroxylgruppen nicht in jedem Fall explizit dargestellt. Die nachfolgenden Beispiele sollen die vorliegende Erfindung näher erläutern ohne diese zu beschränken:

### Herstellung der Ausgangsverbindungen:

### Beispiel I

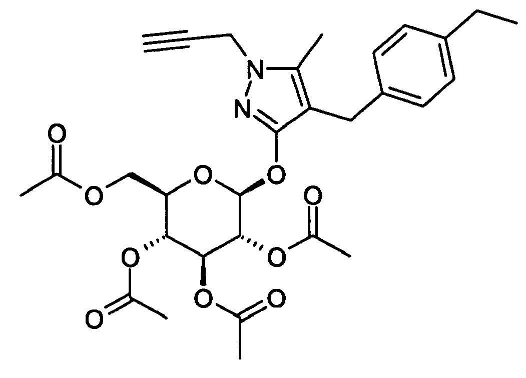

### 1-(2-Propin-1-yl)-3-(2,3,4,6-tetra-O-acetyl-β-D-glucopyranosyloxy)-4-(4-ethylbenzyl)-5-methyl-1 H-pyrazol

600 mg 3-(2,3,4,6-Tetra-O-acetyl-β-D-glucopyranosyloxy)-4-(4-ethylbenzyl)-5-methyl-1*H*-pyrazol (zur Synthese siehe auch WO 01/16147, WO 02/53573, WO 02/68439), 390 mg Cesiumcarbonat und 0.091 ml Propargylbromid werden in 5 ml Dimethylformamid 16 Stunden gerührt. Es werden nochmal 195 mg Cesiumcarbonat und 0.046 ml Propargylbromid zugesetzt und weitere 3 Stunden bei Raumtemperatur gerührt. Das Reaktionsgemisch wird zwischen 25 ml Wasser und 30 ml Essigester verteilt. Die organische Phase wird abgetrennt, getrocknet und eingeengt. Der Rückstand wird durch Chromatographie über eine Kieselgelsäule mit einem Methylenchlorid/Methanol-Gradienten (99:1 bis 95:5) gereinigt.
Ausbeute: 234 mg (36% der Theorie)
Rf-Wert: 0.65 (Kieselgel; Methylenchlorid/Methanol = 15:1)
Massenspektrum (ESI⁺): m/z = 585 [M+H]⁺

Analog Beispiel I werden folgende Verbindung erhalten:

### (1) 1-(2-Butin-1-yl)-3-(2,3,4,6-tetra-O-acetyl-β-D-glucopyranosyloxy)-4-(4-ethylbenzyl)-5-methyl-1 H-pyrazol

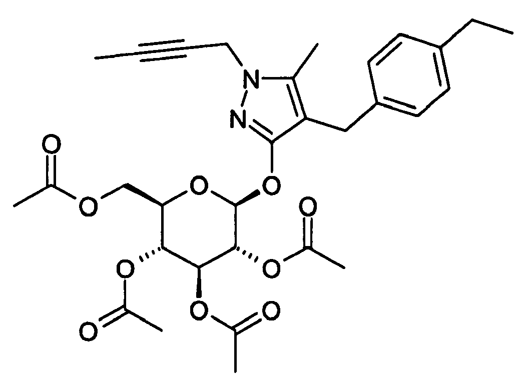

R_{f}-Wert: 0.58 (Kieselgel, Methylenchlorid/Methanol/Cyclohexan = 20:1:2) Massenspektrum (ESI⁺): m/z = 599 [M+H] ⁺

### (2) 1-Isopropyl-3-(2,3,4,6-tetra-O-acetyl-β-D-glucopyranosyloxy)-4-(4-benzyloxybenzyl)-5-methyl-1 H-pyrazol

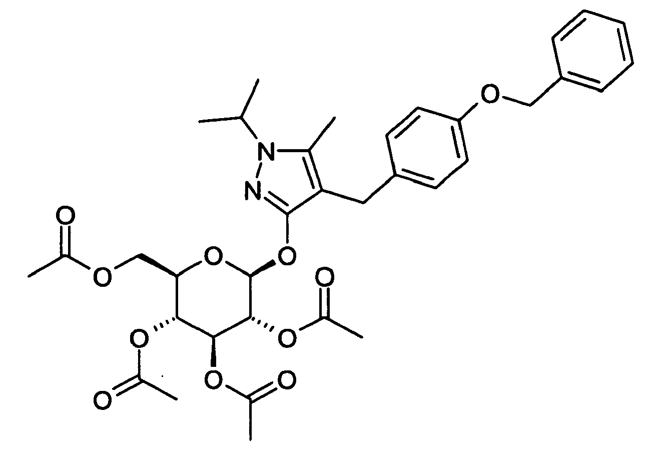

hergestellt aus der Verbindung des Beispiels IV durch Umsetzung mit Isopropyliodid R_{f}-Wert: 0.72 (Kieselgel, Cyclohexan/Essigester = 1:1)
Massenspektrum (ESI⁺): m/z = 667 [M+H] ⁺
Darüberhinaus werden aus dem Reaktionsgemisch teilweise deacetylierte Derivate der Titelverbindung mit R_{f}-Werten von 0.48, 0.33 und 0.22 isoliert (Kieselgel, Cyclohexan/Essigester = 1:1).

### Beispiel II

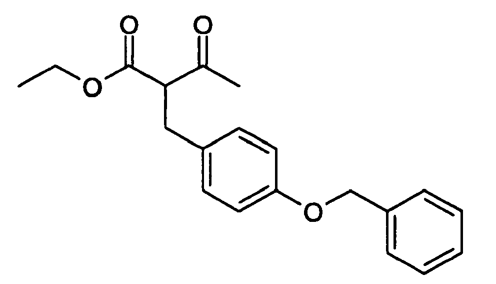

### 2-(4-Benzyloxybenzyl)-acetessigsäure-ethylester

hergestellt durch Behandlung von Acetessigsäureethylester mit Natriumhydrid und anschließender Umsetzung mit 4-Benzyloxybenzylbromid. Die Reaktion wird in Tetrahydrofuran durchgeführt.
R_{f}-Wert: 0.48 (Kieselgel, Cyclohexan/Essigester = 4:1)
Massenspektrum (ESI⁺): m/z = 344 [M+NH₄]⁺

### Beispiel III

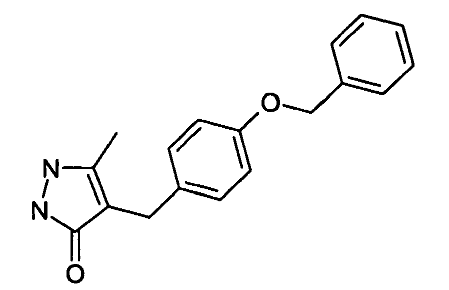

### 1,2-Dihydro-4-(4-benzyloxybenzyl)-5-methyl-pyrazol-3-on

hergestellt aus der Verbindung des Beispiels II durch Umsetzung mit Hydrazin-hydrat in Ethanol
R_{f}-Wert: 0.35 (Kieselgel, Methylenchlorid/Methanol = 9:1)
Massenspektrum (ESI⁻): m/z = 293 [M-H]⁻

### Beispiel IV

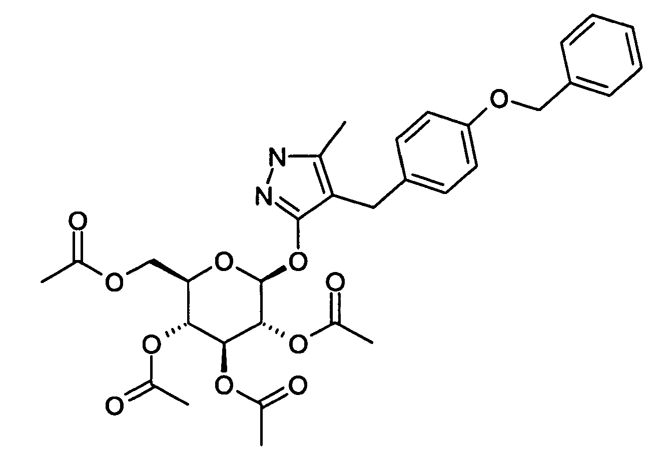

### 3-(2,3,4,6-Tetra-O-acetyl-β-D-glucopyranosyloxy)-4-(4-benzyloxybenzyl)-5-methyl-1H-pyrazol

5.1 g 1,2-Dihydro-4-(4-benzyloxybenzyl)-5-methyl-pyrazol-3-on, 7.13 g 2,3,4,6-Tetra-O-acetyl-alpha-glucopyranosylbromid und 4.78 g Silbercarbonat werden 3 Tage bei 65°C unter Lichtausschluß gerührt. Das Reaktionsgemisch wird über eine Nutsche filtriert, der feste Rückstand mit Methylenchlorid gewaschen und das Filtrat eingeengt. Das Rohprodukt wird durch Chromatographie über eine Kieselgelsäule mit einem Essigester/Cyclohexan-Gradienten (1:1 bis 3:1) gereinigt.
Ausbeute: 4.7 g (43% der Theorie)
Rf-Wert: 0.45 (Kieselgel; Essigester/Cyclohexan = 3:1)
Massenspektrum (ESI⁺): m/z = 625 [M+H]⁺

### Beispiel V

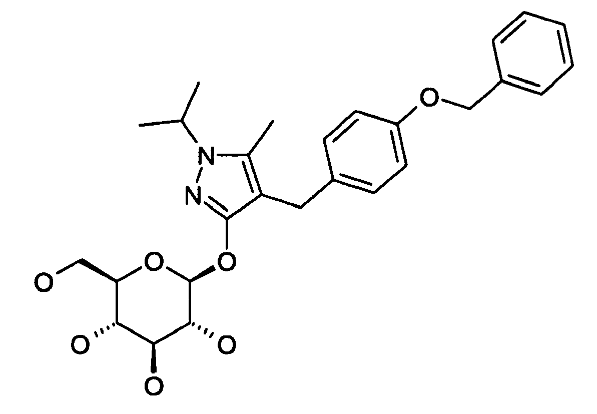

### 1-Isopropyl-3-(β-D-glucopyranosyloxy)-4-(4-benzyloxybenzyl)-5-methyl-1H-pyrazol

wird aus den teilweise deacetylierten Derivaten der Verbindung des Beispiels I(2) durch Behandlung mit Lithiumhydroxid in Tetrahydrofuran/Methanol hergestellt. Rf-Wert: 0.10 (Kieselgel; Methylenchlorid/Methanol = 9:1)
Massenspektrum (ESI⁺): m/z = 499 [M+H]⁺

### Beispiel VI

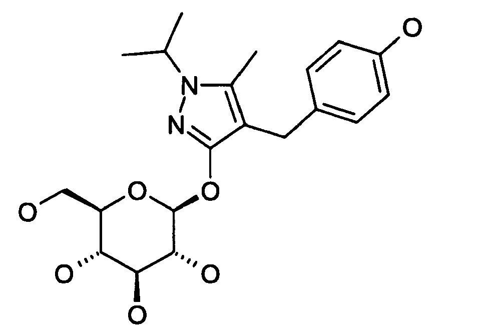

### 1-Isopropyl-3-(β-D-glucopyranosyloxy)-4-(4-hydroxybenzyl)-5-methyl-1H-pyrazol

wird durch katalytische Hydrierung von 1-Isopropyl-3-(β-D-glucopyranosyloxy)-4-(4-benzyloxybenzyl)-5-methyl-1 H-pyrazol in Methanol in Gegenwart von Palladium auf Aktivkohle (10% Pd) bei Raumtemperatur (ca. 20°C) hergestellt.
Rf-Wert: 0.36 (Kieselgel; Methylenchlorid/Methanol = 6:1)
Massenspektrum (ESI⁺): m/z = 409 [M+H]⁺

Analog Beispiel VI wird folgende Verbindung erhalten:

### (1) 1-Isopropyl-3-(2,3,4,6-tetra-O-acetyl-β-D-glucopyranosyloxy)-4-(4-hydroxybenzyl)-5-methyl-1 H-pyrazol

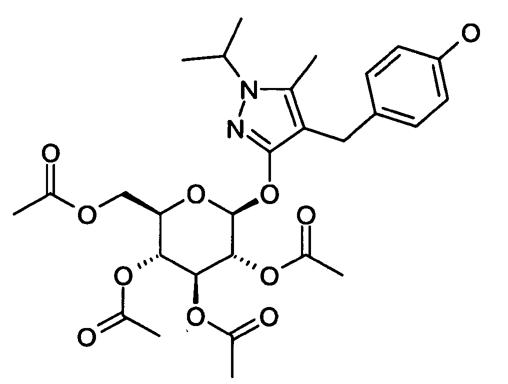

Rf-Wert: 0.30 (Kieselgel; Methylenchlorid/Methanol = 9:1)

### Herstellung der Endverbindungen:

### Beispiel 1

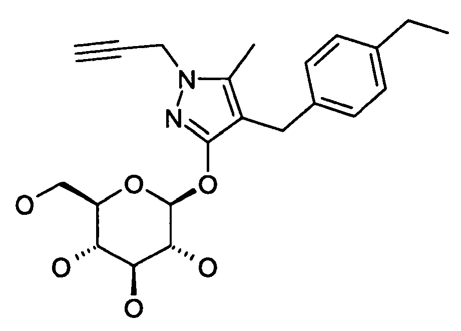

### 1-(2-Propin-1-yl)-3-(β-D-glucopyranosyloxy)-4-(4-ethylbenzyl)-5-methyl-1H-pyrazol (Verbindung nicht Gegenstand der vorliegenden Erfindung)

Eine Lösung von 250 mg 1-(2-Propin-1-yl)-3-(2,3,4,6-tetra-O-acetyl-β-D-glucopyranosyloxy)-4-(4-ethylbenzyl)-5-methyl-1*H*-pyrazol in einem Gemisch aus 1 ml Methanol und 2 ml Tetrahydrofuran wird im Eisbad abgekühlt und mit 1.81 ml einer 1 M wässrigen Lithiumhydroxid-Lösung versetzt und 2 Stunden gerührt. Das Reaktionsgemisch wird mit 5 ml Wasser und 5 ml gesättigter Kochsalzlösung versetzt und mit Essigester extrahiert. Die organische Phase wird abgetrennt, mit gesättigter Kochsalzlösung gewaschen, getrocknet und eingeengt.
Ausbeute: 167 mg (93% der Theorie)
Rf-Wert: 0.17 (Kieselgel; Methylenchlorid/Methanol = 9:1)
Massenspektrum (ESI⁺): m/z = 417 [M+H]⁺

Analog Beispiel 1 wird folgende Verbindung erhalten:

### (1) 1-(2-Butin-1-yl)-3-(β-D-glucopyranosyloxy)-4-(4-ethylbenzyl)-5-methyl-1H-pyrazol (Verbindung nicht Gegenstand der vorliegenden Erfindung)

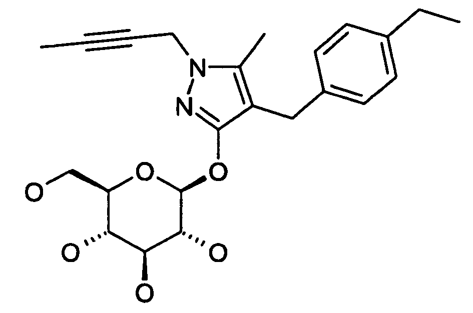

R_{f}-Wert: 0.40 (Kieselgel, Methylenchlorid/Methanol = 9:1)
Massenspektrum (ESI⁺): m/z = 431 [M+H]⁺

### Beispiel 2

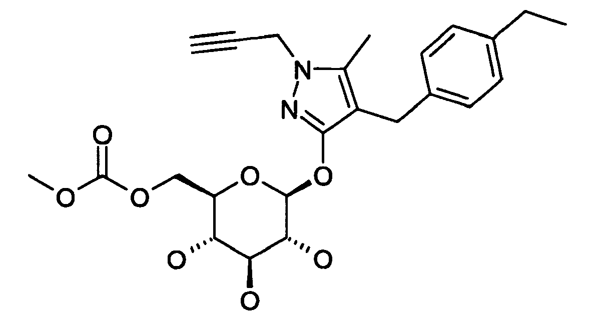

### 1-(2-Propin-1-yl)-3-(6-O-methoxycarbonyl-β-D-glucopyranosyloxy)-4-(4-ethylbenzyl)-5-methyl-1H-pyrazol (Verbindung nicht Gegenstand der vorliegenden Erfindung)

100 mg 1-(2-Propin-1-yl)-3-(β-D-glucopyranosyloxy)-4-(4-ethylbenzyl)-5-methyl-1*H-*pyrazol in 0.5 ml 2,4,6-Collidin werden im Eisbad mit 0.023 ml Chlorameisensäuremethylester versetzt und 6 Stunden gerührt. Zum Reaktionsgemisch werden 3.5 ml 0.1 N Salzsäure gegeben und mit 10 ml Essigester ausgeschüttelt. Die organische Phase wird abgetrennt, mit gesättigter Kochsalzlösung gewaschen und eingeengt. Der Rückstand wird in 20 ml Essigester aufgenommen, nochmals mit 5 ml 0.1 N Salzsäure und gesättigter Kochsalzlösung gewaschen, getrocknet und eingeengt.
Ausbeute: 85 mg (75% der Theorie)
R_{f}-Wert: 0.30 (Kieselgel; Methylenchlorid/Methanol = 9:1)
Massenspektrum (ESI⁺): m/z = 475 [M+H]⁺

Analog Beispiel 2 wird folgende Verbindung erhalten:

### (2) 1-(Tetrahydropyran-4-yl)-3-(6-O-ethoxycarbonyl-β-D-glucopyranosyloxy)-4-(4-methoxybenzyl)-5-methyl-1 H-pyrazol

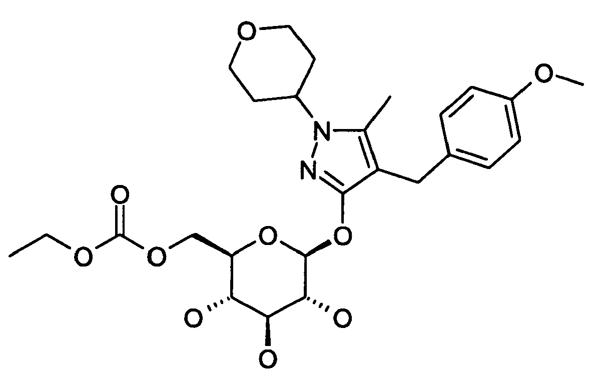

R_{f}-Wert: 0.35 (Kieselgel, Methylenchlorid/Methanol = 9:1)
Massenspektrum (ESI⁺): m/z = 537 [M+H]⁺

### Beispiel 3

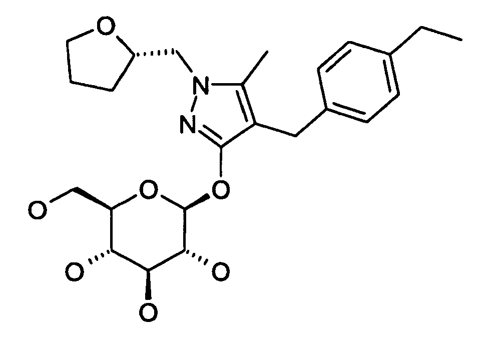

### 1-((S)-Tetrahydrofuran-2-ylmethyl)-3-(β-D-glucopyranosyloxy)-4-(4-ethylbenzyl)-5-methyl-1 H-pyrazol

300 mg 3-(2,3,4,6-Tetra-O-acetyl-β-D-glucopyranosyloxy)-4-(4-ethylbenzyl)-5-methyl-1*H*-pyrazol (zur Synthese siehe auch WO 01/16147, WO 02/53573, WO 02/68439), 1808 mg Cesiumcarbonat und 666 mg (*S*)-p-Toluolsulfonsäure-(tetrahydrofuran-2-ylmethyl)-ester werden in 5 ml Dimethylformamid 5 Stunden bei 100°C gerührt. Das Reaktionsgemisch wird eingeengt, und der Rückstand unter Kühlung im Eisbad mit 0.9 ml Methanol, 1.8 ml Tetrahydrofuran und 1.5 ml 1 N wässriger Lithiumhydroxid-Lösung versetzt. Nach 2 Stunden Rühren wird das Reaktionsgemisch zwischen 20 ml Wasser und 30 ml Essigester verteilt. Die organische Phase wird mit gesättigter Kochsalzlösung gewaschen, getrocknet und eingeengt. Der Rückstand wird durch Chromatographie über eine Kieselgelsäule mit Methylenchlorid/Methanol (9:1) gereinigt.
Ausbeute: 128 mg (50% der Theorie)
Rf-Wert: 0.40 (Kieselgel, Methylenchlorid/Methanol = 7:1)
Massenspektrum (ESI⁺): m/z = 463 [M+H]⁺

Analog Beispiel 3 werden folgende Verbindungen erhalten:

### (3) 1-((S)-Tetrahydrofuran-3-yl)-3-(β-D-glucopyranosyloxy)-4-(4-ethylbenzyl)-5-methyl-1 H-pyrazol

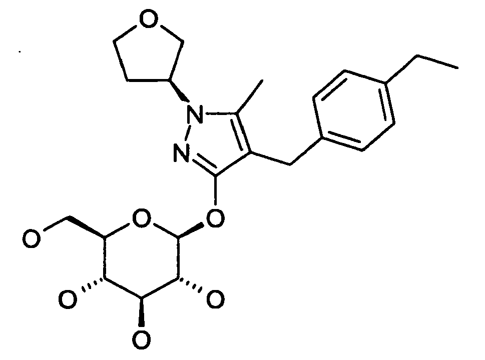

(*R*)-p-Toluolsulfonsäure-(tetrahydrofuran-3-yl)-ester kann als Alkylierungsmittel verwendet werden.
R_{f}-Wert: 0.40 (Kieselgel, Methylenchlorid/Methanol = 7:1)
Massenspektrum (ESI⁺): m/z = 449 [M+H] ⁺

### (4) 1-(Tetrahydropyran-4-yl)-3-(β-D-glucopyranosyloxy)-4-(4-ethylbenzyl)-5-methyl-1 H-pyrazol

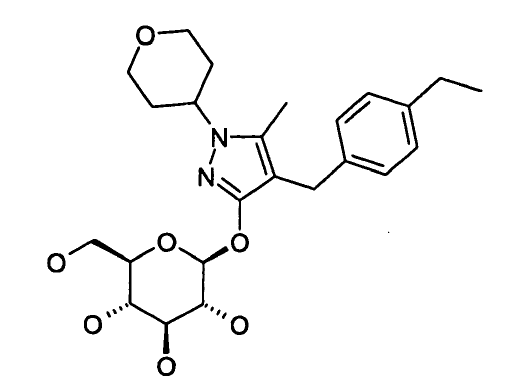

p-Toluolsulfonsäure-(tetrahydropyran-4-yl)-ester kann als Alkylierungsmittel verwendet werden.
R_{f}-Wert: 0.40 (Kieselgel, Methylenchlorid/Methanol = 7:1)
Massenspektrum (ESI⁺): m/z = 463 [M+H] ⁺

### (5) 1-(Tetrahydropyran-4-yl)-3-(β-D-glucopyranosyloxy)-4-(4-methoxybenzyl)-5-methyl-1 H-pyrazol

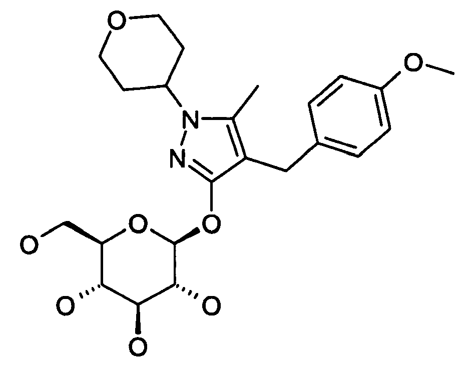

Das Ausgangsmaterial ist beispielsweise 3-(2,3,4,6-Tetra-O-acetyl-β-D-glucopyranosyloxy)-4-(4-methoxybenzyl)-5-methyl-1H-pyrazol, wie es analog in der WO 01/16147 oder WO 02/53573 beschrieben ist. P-Toluolsulfonsäure-(tetrahydropyran-4-yl)-ester kann als Alkylierungsmittel verwendet werden.
R_{f}-Wert: 0.20 (Kieselgel, Methylenchlorid/Methanol = 9:1)
Massenspektrum (ESI⁺): m/z = 465 [M+H] ⁺

Analog den vorstehend genannten Beispielen und anderen literaturbekannten Verfahren werden auch folgende Verbindungen hergestellt:

| | |
|---|---|
| (10) | 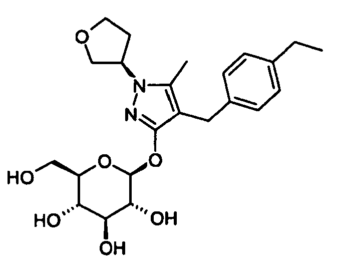 |
| (11) | 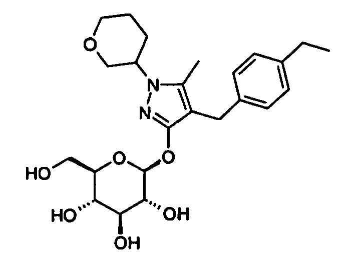 |
| (12) | 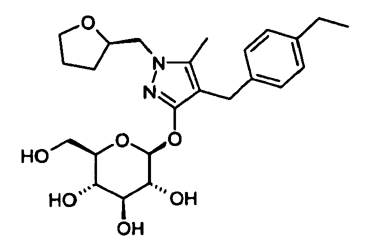 |
| (13) | 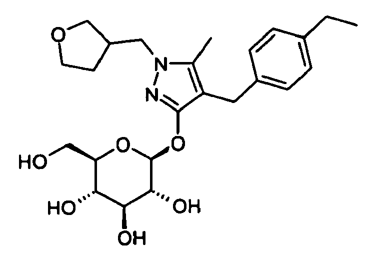 |
| (14) | 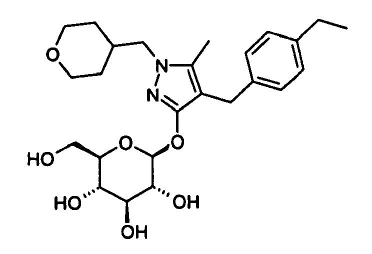 |
| (15) | 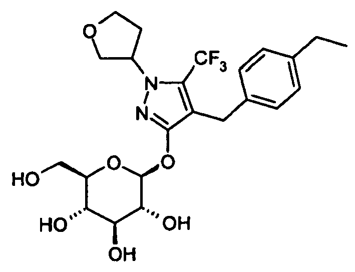 |
| (16) | 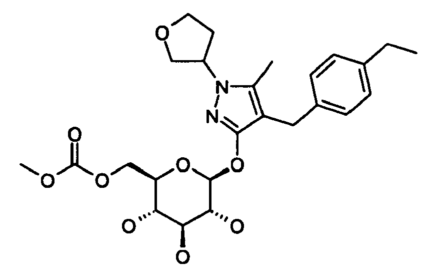 |
| (17) | 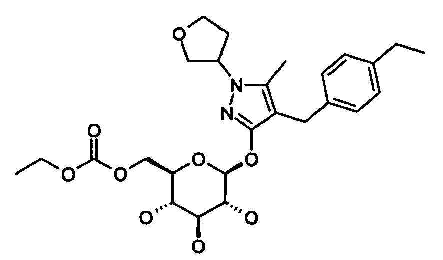 |
| (18) | 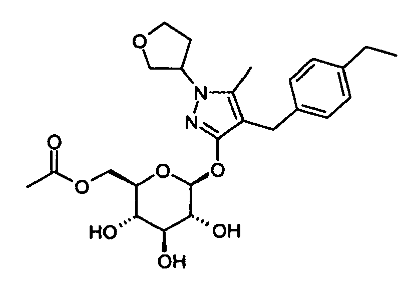 |
| (19) | 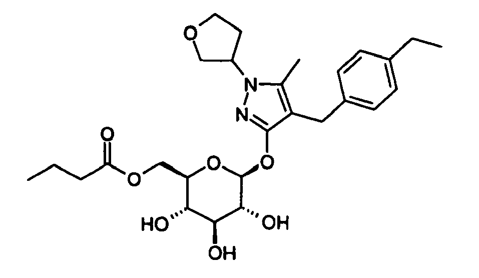 |
| (20) | 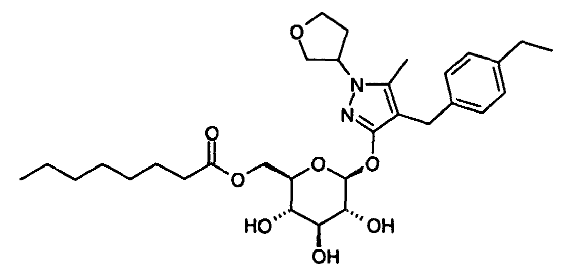 |
| (21) | 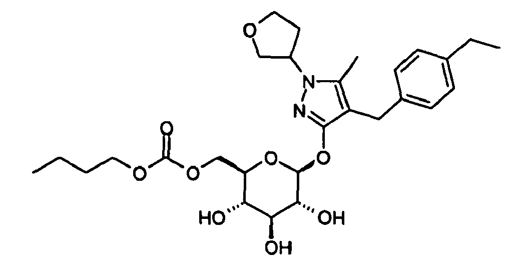 |
| (22) | 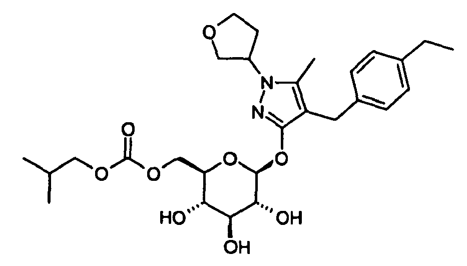 |
| (23) | 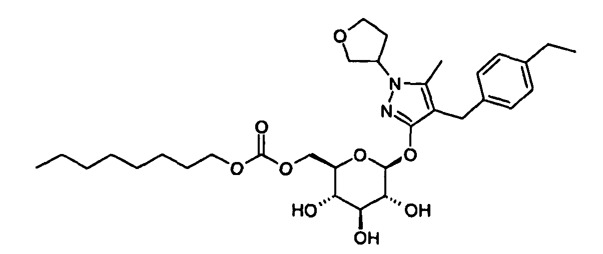 |
| (24) | 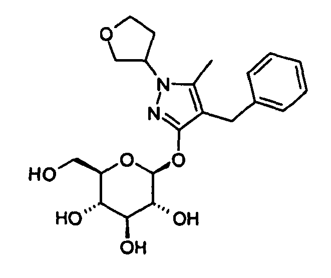 |
| (25) | 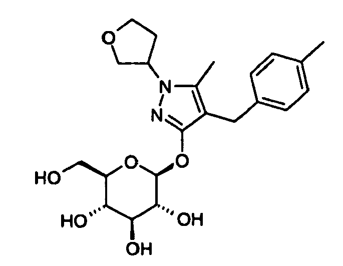 |
| (26) | 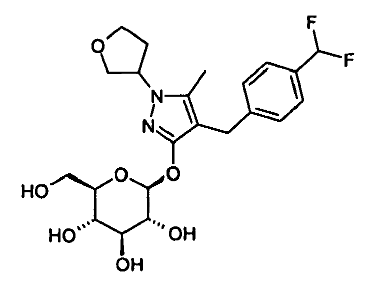 |
| (27) | 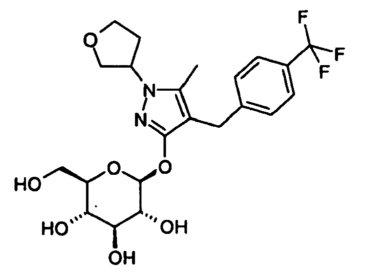 |
| (28) | 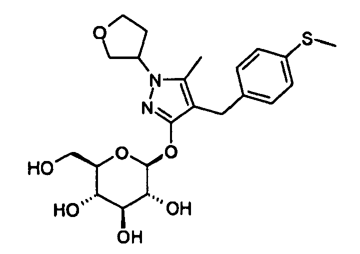 |
| (29) | 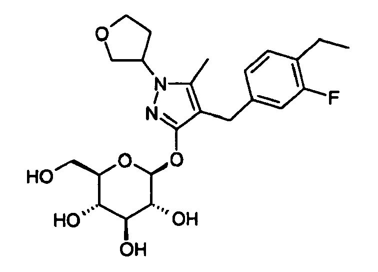 |
| (30) | 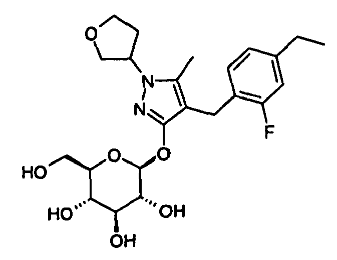 |
| (31) | 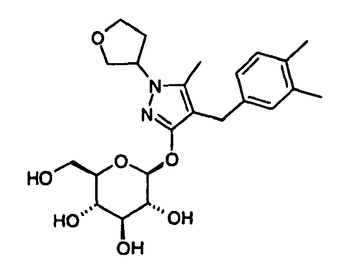 |
| (32) | 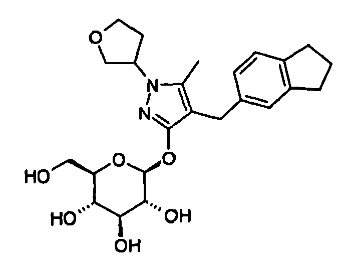 |
| (33) | 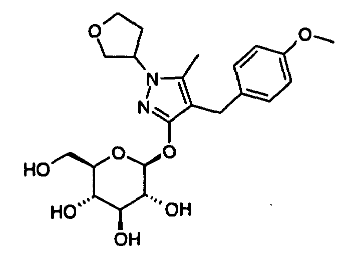 |
| (34) | 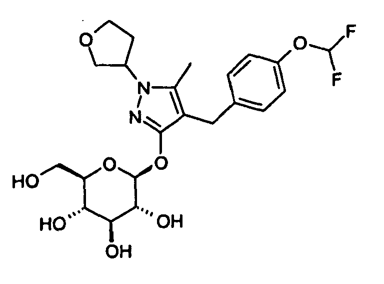 |
| (35) | 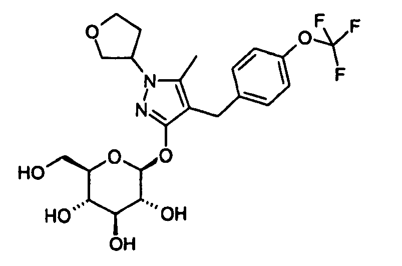 |
| (36) | 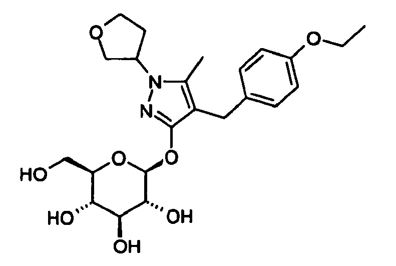 |
| (37) | 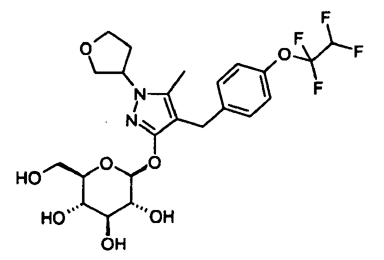 |
| (38) | 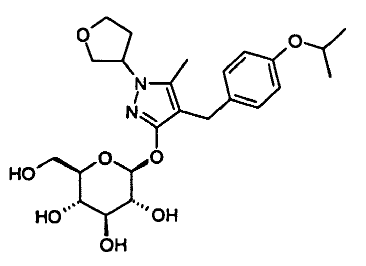 |
| (39) | 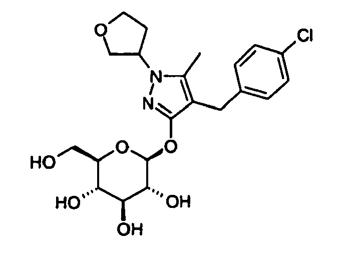 |
| (40) | 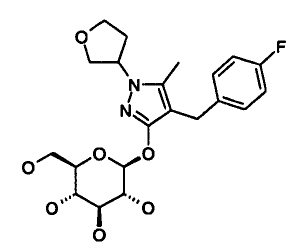 |
| (41) | 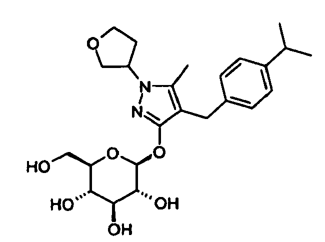 |
| (42) | 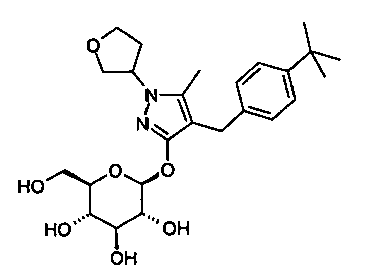 |
| (43) | 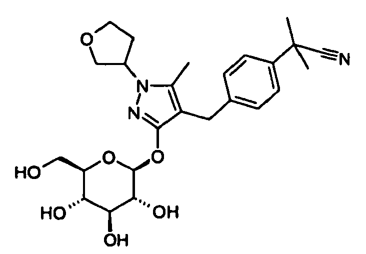 |
| (44) | 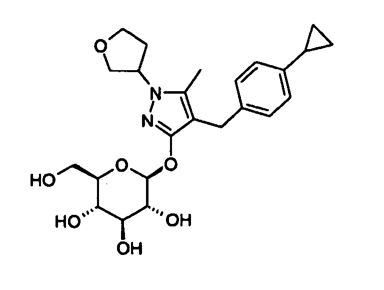 |
| (45) | 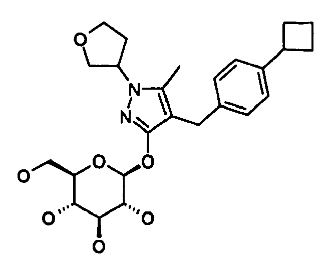 |
| (46) | 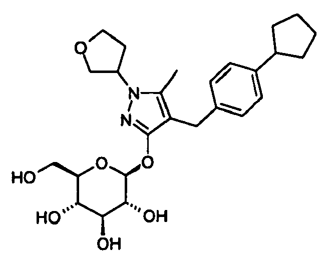 |
| (47) | 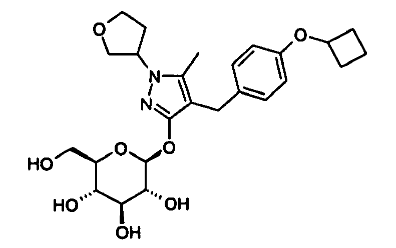 |
| (48) | 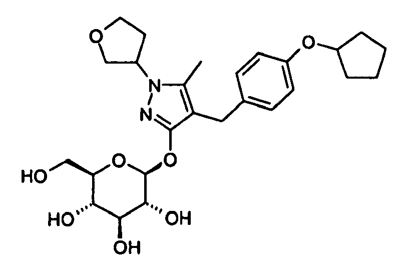 |
| (49) | 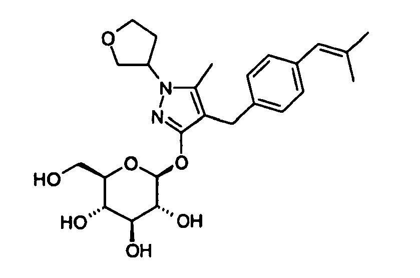 |
| (50) | 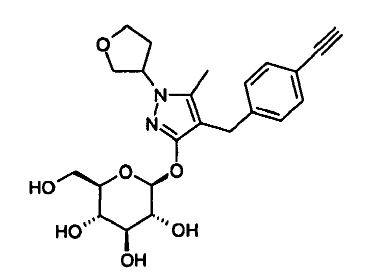 |
| (51) | 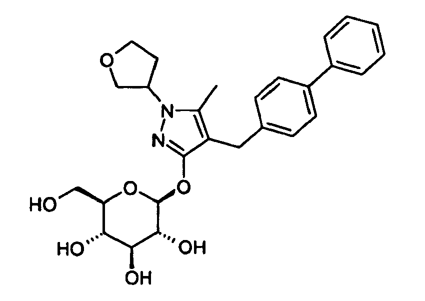 |
| (52) | 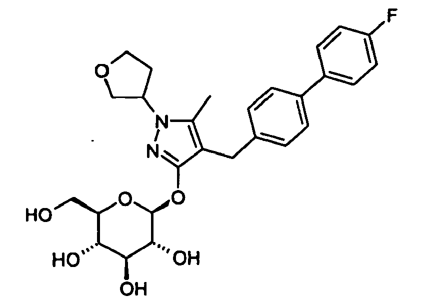 |
| (53) | 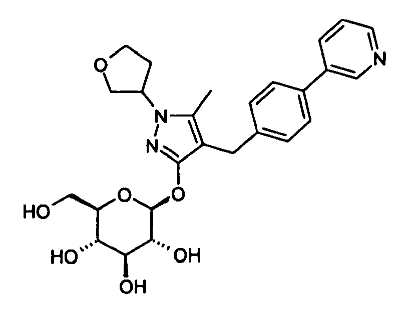 |
| (54) | 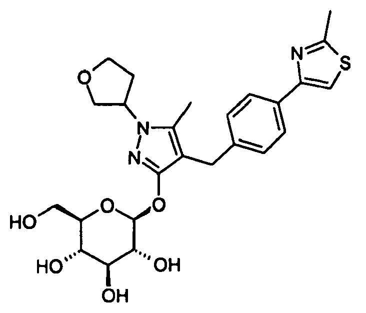 |
| (55) | 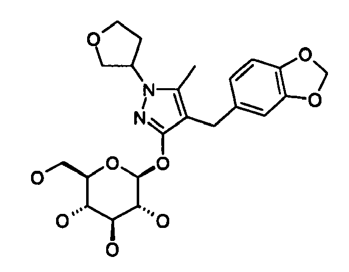 |
| (56) | 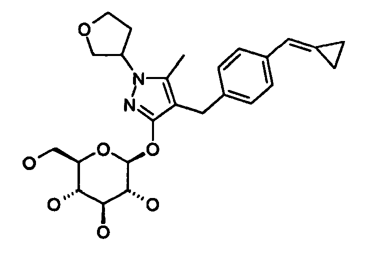 |
| (57) | 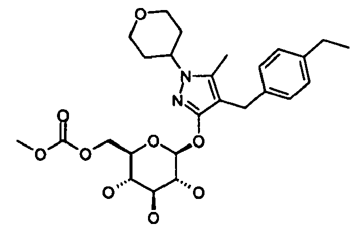 |
| (58) | 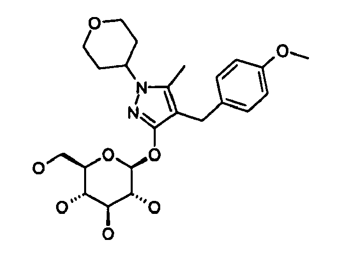 |
| (59) | 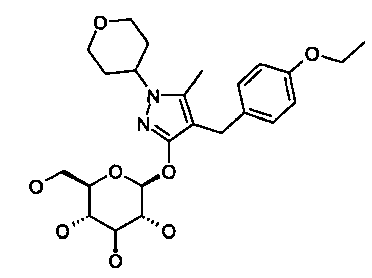 |
| (60) | 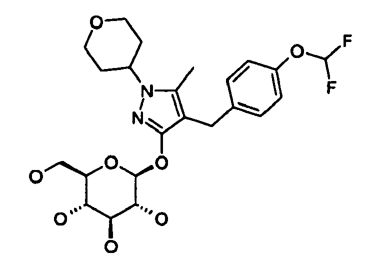 |
| (61) | 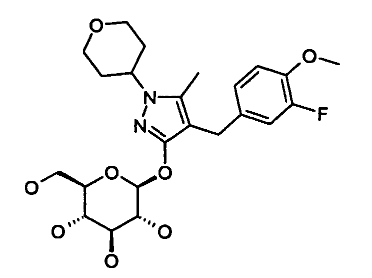 |
| (62) | 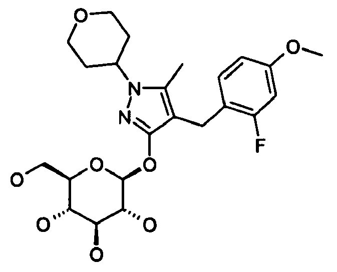 |
| (63) | 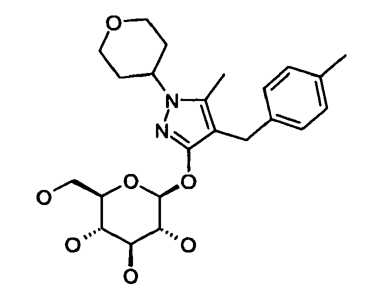 |
| (64) | 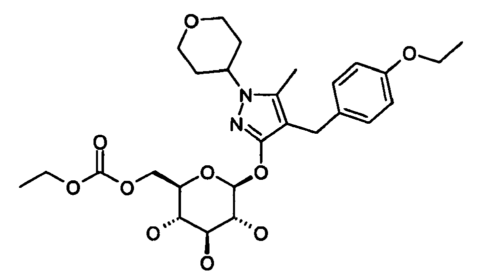 |
| (65) | 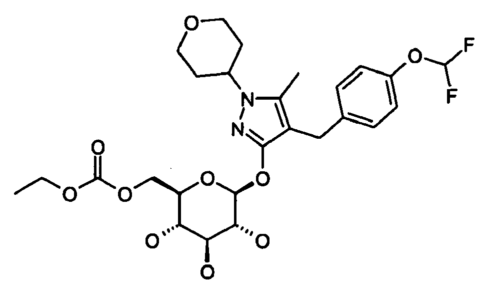 |
| (66) | 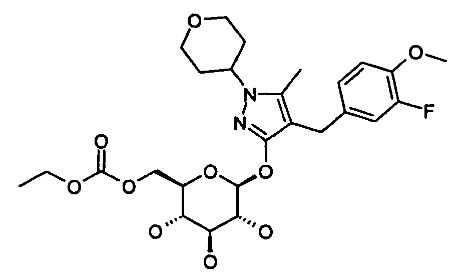 |
| (67) | 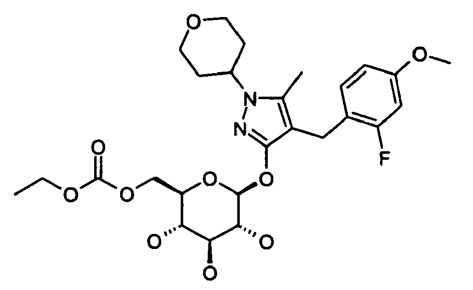 |
| (68) | 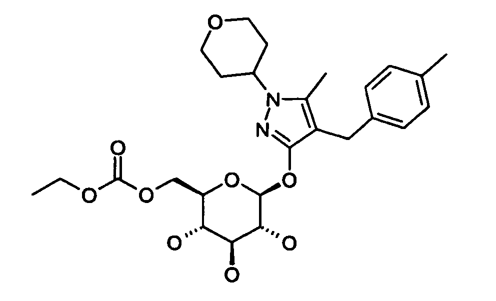 |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

### Tabletten mit 100 mg Wirksubstanz

### Zusammensetzung:

| 1 | Tablette enthält: | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die pressfertige Mischung wird zu Tabletten verarbeitet.

| | | |
|---|---|---|
| Tablettengewicht: | 220 mg | |
| Durchmesser: | 10 mm, | biplan mit beidseitiger Facette |
| | | und einseitiger Teilkerbe. |

### Beispiel B

### Tabletten mit 150 mg Wirksubstanz

### Zusammensetzung:

| 1 | Tablette enthält: | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel C

### Hartgelatine-Kapseln mit 150 mg Wirksubstanz

### Zusammensetzung:

| 1 | Kapsel enthält: | |
|---|---|---|
| | Wirkstoff | 150.0 mg |
| | Maisstärke getr. ca. | 180.0 mg |
| | Milchzucker pulv. ca. | 87.0 mg |
| | Magnesiumstearat | 3.0 mg |
| | ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel D

### Suppositorien mit 150 mg Wirksubstanz

### Zusammensetzung:

| 1 | Zäpfchen enthält: | |
|---|---|---|
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel E

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel F

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. Glucopyranosyloxy-pyrazole der allgemeinen Formel 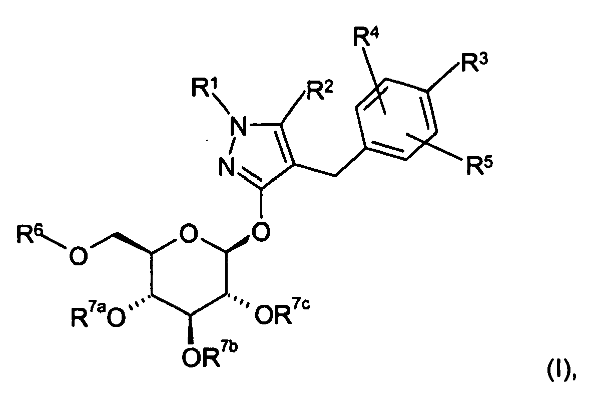 in denen
R¹ Tetrahydrofuran-3-yl, Tetrahydropyran-3-yl, Tetrahydropyran-4-yl, Tetrahydrofuranyl-C₁₋₃-alkyl-, Tetrahydropyranyl-C₁₋₃-alkyl-, Pyrrolidin-3-yl-, Piperidin-3-yl-, Piperidin-4-yl-, oder
eine Pyrrolidin-3-yl-, Piperidin-3-yl- oder Piperidin-4-yl-Gruppe bedeutet, wobei in den drei zuletzt genannten Gruppen das Stickstoffatom mit einer C₁₋₄-Alkyl-, Formyl-, C₁₋₄-Alkyl-carbonyl-, C₁₋₄-Alkylsulfonyl-, Cyan-, Aminocarbonyl-, (C₁₋₄-Alkyl)-aminocarbonyl-, Di-(C₁₋₄-alkyl)-aminocarbonyl- oder (C₁₋₄-Alkyl)-oxycarbonyl-Gruppe substituiert sein kann, und
R² C₁₋₄-Alkyl, durch 1 bis 3 Fluoratome substituiertes C₁₋₄-Alkyl, oder C₃₋₆-Cycloalkyl bedeutet, und
R³ Wasserstoff, Fluor, Chlor, Brom, Iod, C₁₋₆-Alkyl, C₂₋₆-Alkenyl, C₂₋₆-Alkinyl, C₃₋₆-Cycloalkyl, C₃₋₆-Cycloalkylidenmethyl, C₁₋₆-Alkoxy, C₃₋₆-Cycloalkyl-oxy, C₃₋₆-Cycloalkyl-C₁₋₃-alkoxy, Aryl, Aryl-C₁₋₃-alkyl, Heteroaryl, Heteroaryl-C₁₋₃-alkyl, Aryloxy, Aryl-C₁₋₃-alkyl-oxy, durch 1 bis 3 Fluoratome substituiertes Methyl oder Methoxy, durch 1 bis 5 Fluoratome substituiertes C₂₋₄-Alkyl oder C₂₋₄-Alkoxy, durch eine Cyangruppe substituiertes C₁₋₄-Alkyl, durch eine Hydroxy- oder C₁₋₃-Alkyloxygruppe substituiertes C₁₋₄₋Alkyl, Cyan-, Carboxy-, C₁₋₃-Alkoxycarbonyl-, Aminocarbonyl-, (C₁₋₃-Alkylamino)carbonyl-, Di-(C₁₋₃-alkyl)aminocarbonyl-, Pyrrolidin-1-ylcarbonyl-, Piperidin-1-ylcarbonyl-, Morpholin-4-ylcarbonyl-, Piperazin-1-yl-carbonyl-, 4-(C₁₋₃-Alkyl)-piperazin-1-ylcarbonyl-, Nitro-, Amino-, C₁₋₃-Alkylamino-, Di-(C₁₋₃-alkyl)amino-, (C₁₋₄-Alkyl)carbonylamino-, C₁₋₄-Alkylsulfonylamino, Arylsulfonylamino, Aryl-C₁₋₃-alkylsulfonylamino, C₁₋₄-Alkylsulfanyl-, C₁₋₄-Alkylsulfinyl-, C₁₋₄-Alkylsulfonyl, Arylsulfenyl-, Arylsulfinyl- oder Arylsulfonyl- bedeutet, oder
eine Bedeutung ausgewählt aus der Gruppe A besitzt, und
R⁴ und R⁵, die gleich oder verschieden sein können, Wasserstoff, Fluor, Chlor, Brom, C₁₋₃-Alkyl, C₁₋₃-Alkoxy, durch 1 bis 3 Fluoratome substituiertes Methyl- oder Methoxy bedeuten, oder
R³ zusammen mit R⁴, sofern diese an benachbarte Kohlenstoffatome gebunden sind, auch eine geradkettige C₃₋₅-Alkylengruppe, eine Methylendioxygruppe oder Difluormethylendioxy bedeutet, und
R⁶, R^{7a} R^{7b}, R^{7c} unabhängig voneinander eine Bedeutung ausgwählt aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkylfcarbonyl besitzen,
A ausgewählt aus der Gruppe bestehend aus Tetrahydrofuran-3-yloxy-, Tetrahydropyran-3-yloxy-, Tetrahydropyran-4-yloxy-, Tetrahydrofuranyl-C₁₋₃-alkoxy-, Tetrahydropyranyl-C₁₋₃-alkoxy-, Pyrrolidin-3-yloxy-, Piperidin-3-yloxy-, Piperidin-4-yloxy-Gruppe, sowie
Pyrrolidin-3-yloxy-, Piperidin-3-yloxy- und Piperidin-4-yloxy-, wobei in den drei zuletzt genannten Gruppen das Stickstoffatom mit C₁₋₄-Alkyl-, Formyl-, C₁₋₄-Alkyl-carbonyl-, C₁₋₄-Alkylsulfonyl-, Cyan-, Aminocarbonyl-, (C₁₋₄-Alkyl)-aminocarbonyl-, Di-(C₁₋₄-alkyl)-aminocarbonyl- oder (C₁₋₄-Alkyl)-oxycarbonyl-substituiert sein kann,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor, Chlor, Brom, Iod, C₁₋₃-Alkyl, Difluormethyl, Trifluormethyl, C₁₋₃-Alkoxy, Difluormethoxy, Trifluormethoxy oder Cyan bedeutet,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen durch Rₕ mono- oder disubstituiert sein können, wobei die Substituenten gleich oder verschieden sein können und Rₕ wie vorstehend definiert ist,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

2. Glucopyranosyloxy-pyrazole der allgemeinen Formel I gemäß Anspruch 1, in denen
R¹ Tetrahydrofuran-3-yl, Tetrahydropyran-3-yl, Tetrahydropyran-4-yl, Tetrahydrofuranylmethyl oder Tetrahydropyranylmethyl bedeutet, und
R² Methyl- oder Trifluormethyl bedeutet,
R³ Wasserstoff, Fluor, Chlor, Methyl, Ethyl, Isopropyl, tert.-Butyl, 2-Cyan-2-propyl, Difluormethyl, Trifluormethyl, Cyclopropyl, Cyclobutyl, Cyclopentyl, Methoxy, Ethoxy, Isopropoxy, Difluormethoxy, Trifluormethoxy, 1,1,2,2-Tetrafluorethoxy, Cylopropyl-oxy, Cyclobutyl-oxy, Cyclopentyl-oxy, Tetrahydrofuran-3-yloxy, Tetrahydropyran-3-yloxy, Tetrahydropyran-4-yloxy, Tetrahydrofuranylmethyloxy, Tetrahydropyranylmethyloxy, Methylsulfanyl, 2-Methyl-1-propen-1-yl, Cyclopropylidenmethyl-, Ethinyl, Phenyl-, Fluorphenyl, Pyridyl oder Methylthiazolyl bedeutet, und
R⁴ Wasserstoff- oder Fluor bedeutet, oder,
R³ zusammen mit R⁴, sofern diese an benachbarte Kohlenstoffatome gebunden sind, auch 1,3-Propylen-, Methylendioxy- oder Difluormethylendioxy- bedeuten kann, und
R⁵ Wasserstoff und
R⁶ Wasserstoff, (C₁₋₈-Alkyl)oxycarbonyl- oder C₁₋₈-Alkylcarbonyl- bedeutet,
deren Tautomere, deren Stereoisomere, deren Gemische und deren Salze.

3. Glucopyranosyloxy-pyrazole der allgemeinen Formel I gemäß Anspruch 1, in denen
R¹ Tetrahydrofuran-3-yl-, Tetrahydropyran-4-yl- oder Tetrahydrofuran-2-ylmethyl-,
R² Methyl,
R³ Methyl, Ethyl, Methoxy, Ethoxy, Difluormethoxy, Tetrahydrofuran-3-yloxy, Tetrahydropyran-4-yloxy oder Tetrahydrofuranylmethyloxy,
R⁴ Wasserstoff oder Fluor,
R⁵ Wasserstoff und
R⁶ Wasserstoff, Methoxycarbonyl oder Ethoxycarbonyl bedeuten,
deren Tautomere, deren Stereoisomere, deren Gemische und deren Salze.

4. Glucopyranosyloxy-pyrazole der allgemeinen Formel I gemäß Anspruch 1, in denen
R¹ Tetrahydrofuran-3-yl oder Tetrahydropyran-4-yl,
R² Methyl,
R³ Methyl, Ethyl, Methoxy, Ethoxy oder Difluormethoxy,
R⁴ Wasserstoff oder Fluor,
R⁵ Wasserstoff,
R⁶ Wasserstoff, Methoxycarbonyl oder Ethoxycarbonyl und
R^{7a}, R^{7b} und R^{7c} Wasserstoff bedeuten,
deren Tautomere, deren Stereoisomere, deren Gemische und deren Salze.

5. Verbindungen der allgemeinen Formel I gemäß Anspruch 1 ausgewählt aus der Gruppe bestehend aus
(a) 1-((S)-Tetrahydrofuran-3-yl)-3-(β-D-glucopyranosyloxy)-4-(4-ethylbenzyl)-5-methyl-1 H-pyrazol,
(b) 1-(Tetrahydropyran-4-yl)-3-(β-D-glucopyranosyloxy)-4-(4-ethylbenzyl)-5-methyl-1 H-pyrazol,
(c) 1-(Tetrahydropyran-4-yl)-3-(β-D-glucopyranosyloxy)-4-(4-methoxybenzyl)-5-methyl-1 H-pyrazol,
sowie deren Derivate, in denen R⁶ ein erfindungsgemäße, von Wasserstoff verschiedenen Bedeutung aufweist, insbesondere R⁶ Ethoxycarbonyl oder Methoxycarbonyl bedeutet,
einschließlich deren Tautomere, deren Stereoisomere, deren Gemische und deren Salze.

6. Physiologisch verträgliche Salze der Verbindungen nach mindestens einem der Ansprüche 1 bis 5 mit anorganischen oder organischen Säuren.

7. Arzneimittel, enthaltend eine Verbindung nach einem oder mehreren der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung mindestens einer Verbindung nach einem oder mehreren der Ansprüche 1 bis 5 oder eines physiologisch verträglichen Salzes gemäß Anspruch 6 zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind.

9. Verwendung mindestens einer Verbindung nach einem oder mehreren der Ansprüche 1 bis 5 oder eines physiologisch verträglichen Salzes gemäß Anspruch 6 zur Herstellung eines Arzneimittels, das zur Behandlung oder Prophylaxe von Stoffwechselerkrankungen geeignet ist.

10. Verwendung nach Anspruch 9, **dadurch gekennzeichnet, dass** die Stoffwechserkrankung ausgewählt ist aus der Gruppe bestehend aus Diabetes mellitus Typ 1 und Typ 2, diabetische Komplikationen, metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie.

11. Verwendung mindestens einer Verbindung nach mindestens einem der Ansprüche 1 bis 5 oder eines physiologisch verträglichen Salzes gemäß Anspruch 6 zur Herstellung eines Arzneimittels zur Inhibition des natriumabhängigen Glucose-Cotransporters SGLT.

12. Verwendung mindestens einer Verbindung nach mindestens einem der Ansprüche 1 bis 5 oder eines physiologisch verträglichen Salzes gemäß Anspruch 6 zur Herstellung eines Arzneimittels zum Verhindern der Degeneration von pankreatischen beta-Zellen und/oder zum Verbessern und/oder Wiederherstellen der Funktionalität von pankreatischen beta-Zellen.

13. Verwendung mindestens einer Verbindung nach mindestens einem der Ansprüche 1 bis 5 oder eines physiologisch verträglichen Salzes gemäß Anspruch 6 zur Herstellung von Diuretika und/oder Antihypertensiva.

14. Verfahren zur Herstellung eines Arzneimittels gemäß Anspruch 8, **dadurch gekennzeichnet, dass** auf nicht-chemischem Wege eine Verbindung nach mindestens einem der Ansprüche 1 bis 5 oder ein physiologisch verträgliches Salz gemäß Anspruch 6 in einen oder mehrere inerte Trägerstoffe und/oder Verdünnungsmittel eingearbeitet wird.

15. Verfahren zur Herstellung der Verbindungen der allgemeinen Formel I gemäß den Ansprüchen 1 bis 5, **dadurch gekennzeichnet, dass**
a) zur Herstellung von Verbindungen der allgemeinen Formel 1, in der R¹ eine der in den Ansprüchen 1 bis 5 erwähnten Gruppen,
eine Verbindung der allgemeinen Formel 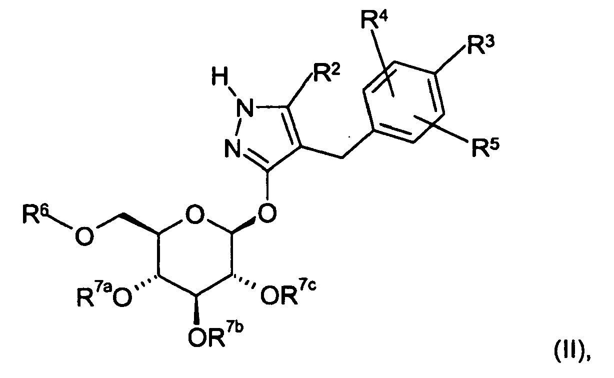 in der
R² bis R⁶ sowie R^{7a}, R^{7b} , R^{7c} wie in den Ansprüchen 1 bis 5 erwähnt definiert sind, mit einer Verbindung der allgemeinen Formel
Z¹ - R^{1'} (III),
in der
R^{1'} die eingangs für R¹ erwähnten Bedeutungen besitzt und Z¹ eine Austrittsgruppe darstellt, umgesetzt wird, oder
b) zur Herstellung von Verbindungen der allgemeinen Formel 1, in der R³ gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die gemäß Anspruch 1 definiert ist, darstellt,
eine Verbindung der allgemeinen Formel 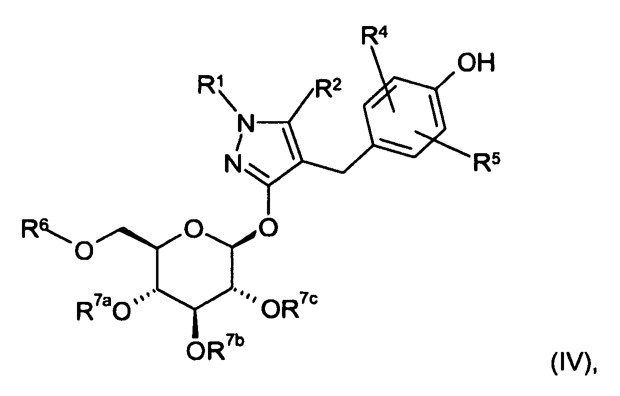 in der
R¹, R² und R⁴ bis R⁶ sowie R^{7a}, R^{7b} , R^{7c} wie in Anspruch 1 definiert sind, mit einer Verbindung der allgemeinen Formel
Z² - R³' (V),
in der
R^{3'} gegebenenfalls substituiertes C₁₋₆-Alkoxy, C₃₋₆-Cycloalkyl-oxy, C₃₋₅-Cycloalkyl-C₁₋₃-alkoxy oder eine Bedeutung ausgewählt aus der Gruppe A, die wie vorstehend erwähnt definiert ist, darstellt, und Z² eine Austrittsgruppe, vorzugsweise ein Halogenatom, beispielsweise ein Chlor- oder Bromatom, eine Sulfonyloxygruppe, beispielsweise eine Methansulfonyloxy- oder p-Toluolsulfonyloxygruppe oder eine Hydroxygruppe darstellt, umgesetzt wird und gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, übergeführt wird.

## Claims

1. Glucopyranosyloxy-pyrazoles of general formula 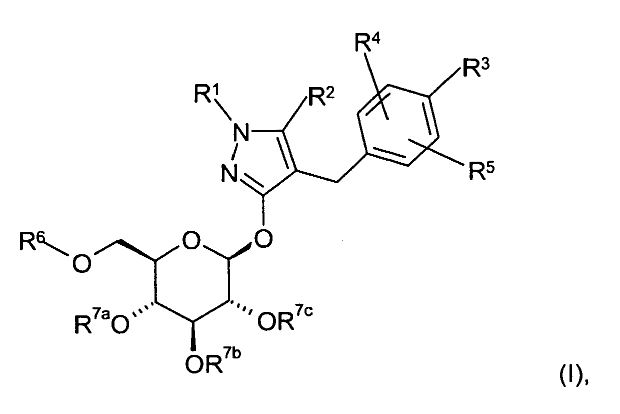 wherein
R¹ denotes tetrahydrofuran-3-yl, tetrahydropyran-3-yl, tetrahydropyran-4-yl, tetrahydrofuranyl-C₁₋₃-alkyl, tetrahydropyranyl-C₁₋₃-alkyl, pyrrolidin-3-yl, piperidin-3-yl, piperidin-4-yl, or
a pyrrolidin-3-yl, piperidin-3-yl or piperidin-4-yl group, while in the latter three groups the nitrogen atom may be substituted by a C₁₋₄-alkyl, formyl, C₁₋₄-alkyl-carbonyl, C₁₋₄-alkylsulphonyl, cyano, aminocarbonyl, (C₁₋₄-alkyl)-aminocarbonyl, di-(C₁₋₄-alkyl)-aminocarbonyl or (C₁₋₄-alkyl)-oxycarbonyl group, and
R² denotes C₁₋₄-alkyl, a C₁₋₄-alkyl group substituted by 1 to 3 fluorine atoms, or C₃₋₆-cycloalkyl, and
R³ denotes hydrogen, fluorine, chlorine, bromine, iodine, C₁₋₆-alkyl, C₂₋₆-alkenyl, C₂₋₆-alkynyl, C₃₋₆-cycloalkyl, C₃₋₆-cycloalkylidenemethyl, C₁₋₆-alkoxy, C₃₋₆-_{C}ycloalkyl-oxy, C₃₋₆-Cycoalkyl-C₁₋₃-alkoxy, aryl, aryl-C₁₋₃-alkyl, heteroaryl, heteroaryl-C₁₋₃-alkyl, aryloxy, aryl-C₁₋₃-alkyl-oxy, a methyl or methoxy group substituted by 1 to 3 fluorine atoms, a C₂₋₄-alkyl or C₂₋₄-alkoxy group substituted by 1 to 5 fluorine atoms, a C₁₋₄-alkyl group substituted by a cyano group, a C₁₋₄-alkyl group substituted by a hydroxy or C₁₋₃-alkyloxy group, or cyano, carboxy, C₁₋₃-alkoxycarbonyl, aminocarbonyl, (C₁₋₃-alkylamino)carbonyl, di-(C₁₋₃-alkyl)aminocarbonyl, pyrrolidin-1-ylcarbonyl, piperidin-1-ylcarbonyl, morpholin-4-ylcarbonyl, piperazin-1-yl-carbonyl, 4-(C₁-₃-alkyl)-piperazin-1-ylcarbonyl, nitro, amino, C₁₋₃-alkylamino, di-(C₁₋₃-alkyl)amino, (C₁₋₄-alkyl)carbonylamino, C₁₋₄-alkylsulphonylamino, arylsulphonylamino, aryl-C₁₋₃-alkylsulphonylamino, C₁₋₄-alkylsulphanyl, C₁₋₄-alkylsulphinyl, C₁₋₄-alkylsulphonyl, arylsulphenyl, arylsulphinyl or arylsulphonyl, or
has a meaning selected from the group A, and
R⁴ and R⁵, which may be identical or different, represent hydrogen, fluorine, chlorine, bromine, C₁₋₃-alkyl, C₁₋₃-alkoxy, a methyl or methoxy group substituted by 1 to 3 fluorine atoms, or
R³ together with R⁴, if they are bound to adjacent carbon atoms, may also represent a straight-chain C₃₋₅-alkylene, a methylenedioxy group or difluoromethylenedioxy, and
R⁶, R^{7a} R^{7b}, R^{7c} independently of one another have a meaning selected from the group hydrogen, (C₁₋₁₈-alkyl)carbonyl, (C₁₋₁₈-alkyl)oxycarbonyl, arylcarbonyl and aryl-(C₁₋₃-alkyl)-carbonyl,
A is selected from the group consisting of tetrahydrofuran-3-yloxy, tetrahydropyran-3-yloxy, tetrahydropyran-4-yloxy, tetrahydrofuranyl-C₁₋₃-alkoxy, tetrahydropyranyl-C₁₋₃-alkoxy, pyrrolidin-3-yloxy, piperidin-3-yloxy, piperidin-4-yloxy group, and
pyrrolidin-3-yloxy, piperidin-3-yloxy and piperidin-4-yloxy, while in the latter three groups the nitrogen atom may be substituted by C₁₋₄-alkyl, formyl, C₁₋₄-alkyl-carbonyl, C₁₋₄-alkylsulphonyl, cyano, aminocarbonyl, (C₁₋₄-alkyl)-aminocarbonyl, di-(C₁₋₄-alkyl)-aminocarbonyl or (C₁₋₄-alkyl)-oxycarbonyl,
while the aryl groups mentioned in the definition of the above groups are meant to indicate phenyl or naphthyl groups which may be mono- or disubstituted independently of one another by Rₕ, while the substituents may be identical or different and Rₕ denotes a fluorine, chlorine, bromine, iodine, C₁₋₃-alkyl, difluoromethyl, trifluoromethyl, C₁₋₃-alkoxy, difluoromethoxy, trifluoromethoxy or cyano,
the heteroaryl groups mentioned in the definition of the above-mentioned groups are meant to indicate a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group, wherein one or two methyne groups are replaced by nitrogen atoms,
or an indolyl, benzofuranyl, benzothiophenyl, quinolinyl or isoquinolinyl group, wherein one to three methyne groups are replaced by nitrogen atoms,
while the above-mentioned heteroaryl groups may be mono- or disubstituted by Rₕ, while the substituents may be identical or different and Rₕ is as hereinbefore defined,
while, unless otherwise stated, the above-mentioned alkyl groups may be straight-chain or branched,
the tautomers, the stereoisomers, the mixtures thereof and the salts thereof.

2. Glucopyranosyloxy-pyrazoles of general formula I according to claim 1, wherein
R¹ denotes tetrahydrofuran-3-yl, tetrahydropyran-3-yl, tetrahydropyran-4-yl, tetrahydrofuranylmethyl or tetrahydropyranylmethyl, and
R² denotes methyl or trifluoromethyl,
R³ denotes hydrogen, fluorine, chlorine, methyl, ethyl, isopropyl, tert.-butyl, 2-cyano-2-propyl, difluoromethyl, trifluoromethyl, cyclopropyl, cyclobutyl, cyclopentyl, methoxy, ethoxy, isopropoxy, difluoromethoxy, trifluoromethoxy, 1,1,2,2-tetrafluoroethoxy, cylopropyl-oxy, cyclobutyl-oxy, cyclopentyl-oxy, tetrahydrofuran-3-yloxy, tetrahydropyran-3-yloxy, tetrahydropyran-4-yloxy, tetrahydrofuranylmethyloxy, tetrahydropyranylmethyloxy, methylsulphanyl, 2-methyl-1-propen-1-yl, cyclopropylidenemethyl, ethynyl, phenyl, fluorophenyl, pyridyl or methylthiazolyl, and
R⁴ denotes hydrogen or fluorine, or
R³ together with R⁴, if they are bound to adjacent carbon atoms, may also represent 1,3-propylene, methylenedioxy or difluoromethylenedioxy, and
R⁵ denotes hydrogen and
R⁶ denotes hydrogen, (C₁₋₈-alkyl)oxycarbonyl or C₁₋₈-alkylcarbonyl,
the tautomers, the stereoisomers, the mixtures thereof and the salts thereof.

3. Glucopyranosyloxy-pyrazoles of general formula I according to claim 1, wherein
R¹ denotes tetrahydrofuran-3-yl, tetrahydropyran-4-yl or tetrahydrofuran-2-ylmethyl,
R² denotes methyl,
R³ denotes methyl, ethyl, methoxy, ethoxy, difluoromethoxy, tetrahydrofuran-3-yloxy, tetrahydropyran-4-yloxy or tetrahydrofuranylmethyloxy,
R⁴ denotes hydrogen or fluorine,
R⁵ denotes hydrogen and
R⁶ denotes hydrogen, methoxycarbonyl or ethoxycarbonyl,
the tautomers, the stereoisomers, the mixtures thereof and the salts thereof.

4. Glucopyranosyloxy-pyrazoles of general formula I according to claim 1, wherein
R¹ denotes tetrahydrofuran-3-yl or tetrahydropyran-4-yl,
R² denotes methyl,
R³ denotes methyl, ethyl, methoxy, ethoxy or difluoromethoxy,
R⁴ denotes hydrogen or fluorine,
R⁵ denotes hydrogen,
R⁶ denotes hydrogen, methoxycarbonyl or ethoxycarbonyl and
R^{7a}, R^{7b} and R^{7c} denote hydrogen,
the tautomers, the stereoisomers, the mixtures thereof and the salts thereof.

5. Compounds of general formula I according to claim 1 selected from the group consisting of
(a) 1-((S)-tetrahydrofuran-3-yl)-3-(β-D-glucopyranosyloxy)-4-(4-ethylbenzyl)-5-methyl-1H-pyrazole,
(b) 1-(tetrahydropyran-4-yl)-3-(β-D-glucopyranosyloxy)-4-(4-ethylbenzyl)-5-methyl-1 H-pyrazole,
(c) 1-(tetrahydropyran-4-yl)-3-(β-D-glucopyranosyloxy)-4-(4-methoxybenzyl)-5-methyl-1 H-pyrazole,
and the derivatives thereof wherein R⁶ has a meaning according to the invention other than hydrogen, and in particular R⁶ denotes ethoxycarbonyl or methoxycarbonyl,
including the tautomers, the stereoisomers, the mixtures thereof and the salts thereof.

6. Physiologically acceptable salts of the compounds according to at least one of claims 1 to 5 with inorganic or organic acids.

7. Pharmaceutical composition, containing a compound according to one or more of claims 1 to 5 or a physiologically acceptable salt according to claim 6, optionally together with one or more inert carriers and/or diluents.

8. Use of at least one compound according to one or more of claims 1 to 5 or a physiologically acceptable salt according to claim 6 for preparing a pharmaceutical composition which is suitable for the treatment or prevention of diseases or conditions which can be influenced by inhibiting the sodium-dependent glucose cotransporter SGLT.

9. Use of at least one compound according to one or more of claims 1 to 5 or a physiologically acceptable salt according to claim 6 for preparing a pharmaceutical composition which is suitable for the treatment or prevention of metabolic disorders.

10. Use according to claim 9, **characterised in that** the metabolic disorder is selected from the group consisting of type 1 and type 2 diabetes mellitus, complications of diabetes, metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

11. Use of at least one compound according to at least one of claims 1 to 5 or a physiologically acceptable salt according to claim 6 for preparing a pharmaceutical composition for inhibiting the sodium-dependent glucose cotransporter SGLT.

12. Use of at least one compound according to at least one of claims 1 to 5 or a physiologically acceptable salt according to claim 6 for preparing a pharmaceutical composition for preventing the degeneration of pancreatic beta cells and/or for improving and/or restoring the functionality of pancreatic beta cells.

13. Use of at least one compound according to at least one of claims 1 to 5 or a physiologically acceptable salt according to claim 6 for preparing diuretics and/or antihypertensives.

14. Process for preparing a pharmaceutical composition according to claim 8, **characterised in that** a compound according to at least one of claims 1 to 5 or a physiologically acceptable salt according to claim 6 is incorporated in one or more inert carriers and/or diluents by a non-chemical method.

15. Process for preparing the compounds of general formula I according to claims 1 to 5, **characterised in that**
a) in order to prepare compounds of general formula I wherein R¹ denotes one of the groups mentioned in claims 1 to 5,
a compound of general formula 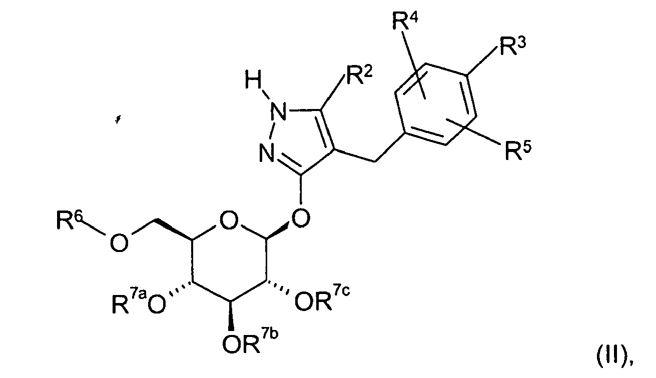 wherein
R² to R⁶ and R^{7a}, R^{7b}, R^{7c} are defined as in claims 1 to 5, is reacted with a compound of general formula
Z¹-R^{1'} (III),
wherein
R^{1'} has the meanings given for R¹ hereinbefore and Z¹ denotes a leaving group, or
b) in order to prepare compounds of general formula I wherein R³ denotes optionally substituted C₁₋₆-alkoxy, C₃₋₆-Cycloalkyl-oxy, C₃₋₅-cycloalkyl-C₁₋₃-alkoxy or has a meaning selected from the group A defined according to claim 1,
a compound of general formula 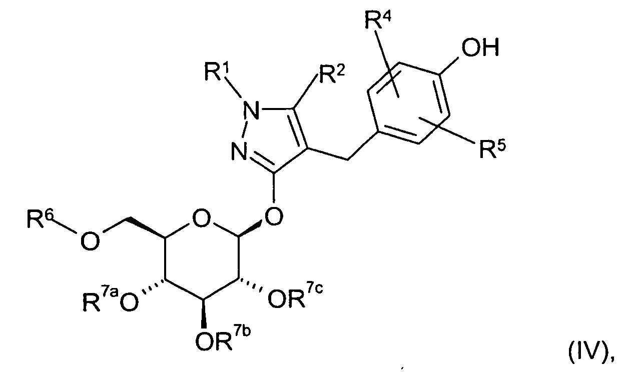 wherein
R¹, R² and R⁴ to R⁶ and R^{7a}, R^{7b} , R^{7c} are defined as in claim 1, is reacted with a compound of general formula
Z²-R^{3'} (V),
wherein
R^{3'} denotes optionally substituted C₁₋₆-alkoxy, C₃₋₆-cycloalkyl-oxy, C₃₋₅-cycloalkyl-C₁₋₃-alkoxy or has a meaning selected from the group A as hereinbefore defined, and Z² denotes a leaving group, preferably a halogen atom, for example a chlorine or bromine atom, a sulphonyloxy group, for example a methanesulphonyloxy or p-toluenesulphonyloxy group or a hydroxy group, and
if desired a compound of general formula I thus obtained wherein R⁶ denotes a hydrogen atom, is converted by acylation into a corresponding acyl compound of general formula I, and/or
if necessary any protecting group used during the reactions described above is cleaved again and/or
if desired a compound of general formula I thus obtained is resolved into its stereoisomers and/or
a compound of general formula I thus obtained is converted into the salts thereof, particularly for pharmaceutical use into the physiologically acceptable salts thereof.

## Revendications

1. Glucopyranosyloxy-pyrazoles de formule générale 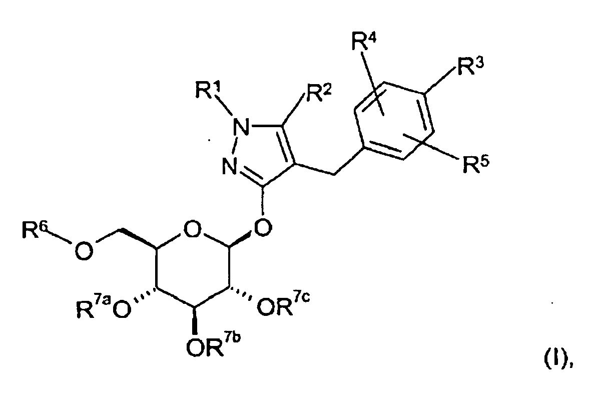 où
R¹ représente tétrahydrofuran-3-yle, tétrahydropyran-3-yle, tétrahydropyran-4-yle, tétrahydrofuranyl-C₁₋₃-alkyle, tétrahydropyranyl-C₁₋₃-alkyle, pyrrolidin-3-yle, pipéridin-3-yle, pipéridin-4-yle ou
un groupe pyrrolidin-3-yle, pipéridin-3-yle ou pipéridin-4-yle, où dans les trois groupes cités en dernier lieu, l'atome d'azote peut être substitué avec un groupe C₁₋₄-alkyle, formyle, C₁₋₄-alkyl-carbonyle, C₁₋₄-alkylsulfonyle, cyano, aminocarbonyle, (C₁₋₄-alkyl)-aminocarbonyle, di-(C₁₋₄-alkyl)-aminocarbonyle ou (C₁₋₄-alkyl)-oxycarbonyle, et
R² représente C₁₋₄-alkyle, C₁₋₄-alkyle substitué par 1 à 3 atomes de fluor, ou C₃₋₆-cycloalkyle, et
R³ représente l'hydrogène, le fluor, le chlore, le brome, l'iode, C₁₋₆-alkyle, C₂₋₆-alcényle, C₂₋₆-alcynyle, C₃₋₆-cycloalkyle, C₃₋₆-cycloalkylidèneméthyle, C₁₋₆-alcoxy, C₃₋₆-cycloalkyl-oxy, C₃₋₆-cycloalkyl-C₁₋₃-alcoxy, aryle, aryl-C₁₋₃-alkyle, hétéroaryle, hétéroaryl-C₁₋₃-alkyle, aryloxy, aryl-C₁₋₃-alkyl-oxy, méthyle ou méthoxy substitué par 1 à 3 atomes de fluor, C₂₋₄-alkyle ou C₂₋₄-alcoxy substitué par 1 à 5 atomes de fluor, C₁₋₄-alkyle substitué par un groupe cyano, C₁₋₄-alkyle substitué par un groupe hydroxy ou C₁₋₃-alkyloxy, cyano, carboxy, C₁₋₃-alcoxycarbonyle, aminocarbonyle, (C₁₋₃-alkylamino)carbonyle, di-(C₁₋₃-alkyl)aminocarbonyle, pyrrolidin-1-ylcarbonyle, pipéridin-1-ylcarbonyle, morpholin-4-ylcarbonyle, pipérazin-1-yl-carbonyle, 4-(C₁₋₃-alkyl)-pipérazin-1-ylcarbonyle, nitro, amino, C₁₋₃-alkylamino, di-(C₁₋₃-alkyl)amino, (C₁₋₄-alkyl)carbonylamino, C₁₋₄-alkylsulfonylamino, arylsylfonylamino, aryl-C₁₋₃-alkylsulfonylamino, C₁₋₄-alkylsulfanyle, C₁₋₄-alkylsulfinyle, C₁₋₄-alkylsulfonyle, arylsulfényle, arylsulfinyle ou arylsulfonyle, ou possède une signification choisie dans le groupe A, et
R⁴ et R⁵, qui peuvent être identiques ou différents, représentent l'hydrogène, le fluor, le chlore, le brome, C₁₋₃-alkyle, C₁₋₃-alcoxy, méthyle ou méthoxy substitué par 1 à 3 atomes de fluor, ou
R³ représente aussi avec R⁴, dans la mesure où ils sont liés à des atomes de carbone voisins, un groupe C₃₋₅-alkylène linéaire, un groupe méthylènedioxy ou difluorométhylènedioxy, et
R⁶, R^{7a} R^{7b}, R^{7c} possèdent indépendamment les uns des autres une signification choisie dans le groupe hydrogène, (C₁₋₁₈-alkyl)carbonyle, (C₁₋₁₈-alkyl)oxycarbonyle, arylcarbonyle et aryl-(C₁₋₃-alkyl)-carbonyle,
A est choisi dans le groupe consistant en tétrahydrofuran-3-yloxy, tétrahydropyran-3-yloxy, tétrahydropyran-4-yloxy, tétrahydrofuranyl-C₁₋₃-alcoxy, tétrahydropyranyl-C₁₋₃-alcoxy, pyrrolidin-3-yloxy, pipéridin-3-yloxy, pipéridin-4-yloxy, ainsi que pyrrolidin-3-yloxy, pipéridin-3-yloxy et pipéridin-4-yloxy, où dans les trois groupes cités en dernier lieu, l'atome d'azote peut être substitué avec C₁₋₄-alkyle, formyle, C₁₋₄-alkyl-carbonyle, C₁₋₄-alkylsulfonyle, cyano, aminocarbonyle, (C₁₋₄-alkyl)-aminocarbonyle, di-(C₁₋₄-alkyl)-aminocarbonyle ou (C₁₋₄-alkyl)-oxycarbonyle,
où, par les groupes aryle cités lors de la définition des groupements cités précédemment, il convient de comprendre des groupes phényle ou naphtyle qui peuvent être mono- ou disubstitués indépendamment les uns des autres par Rₕ, où les substituants peuvent être identiques ou différents et Rₕ représente un fluor, chlore, brome, iode, C₁₋₃-alkyle, difluorométhyle, trifluorométhyle, C₁₋₃-alcoxy, difluorométhoxy, trifluorométhoxy ou cyano,
par les groupes hétéroaryle cités lors de la définition des groupements cités précédemment, il convient de comprendre un groupe pyrrolyle, furanyle, thiényle, pyridyle, indolyle, benzofuranyle, benzothiophényle, quinolinyle ou isoquinolinyle,
ou il convient de comprendre un groupe pyrrolyle, furanyle, thiényle ou pyridyle, où un ou deux groupes méthyne sont remplacés par des atomes d'azote,
ou il convient de comprendre un groupe indolyle, benzofuranyle, benzothiophényle, quinolinyle ou isoquinolinyle, où un à trois groupes méthyne sont remplacés par des atomes d'azote,
où les groupes hétéroaryle cités précédemment peuvent être mono- ou disubstitués par Rₕ, où les substituants peuvent être identiques ou différents et Rₕ est défini comme précédemment,
où, sauf indication contraire, les groupes alkyle cités précédemment peuvent être linéaires ou ramifiés,
leurs tautomères, leurs stéréoisomères, leurs mélanges et leurs sels.

2. Glucopyranosyloxy-pyrazoles de formule générale I selon la revendication 1 où
R¹ représente tétrahydrofuran-3-yle, tétrahydropyran-3-yle, tétrahydropyran-4-yle, tétrahydrofuranylméthyle ou tétrahydropyranylméthyle, et
R² représente méthyle ou trifluorométhyle,
R³ représente l'hydrogène, le fluor, le chlore, méthyle, éthyle, isopropyle, tert-butyle, 2-cyano-2-propyle, difluorométhyle, trifluorométhyle, cyclopropyle, cyclobutyle, cyclopentyle, méthoxy, éthoxy, isopropoxy, difluorométhoxy, trifluorométhoxy, 1,1,2,2-tétrafluoroéthoxy, cyclopropyl-oxy, cyclobutyl-oxy, cyclopentyl-oxy, tétrahydrofuran-3-yloxy, tétrahydropyran-3-yloxy, tétrahydropyran-4-yloxy, tétrahydrofuranyl-méthyloxy, tétrahydropyranyl-méthyloxy, méthylsulfanyle, 2-méthyl-1-propén-1-yle, cyclopropylidèneméthyle, éthynyle, phényle, fluorophényle, pyridyle ou méthylthiazolyle, et
R⁴ représente l'hydrogène ou le fluor, ou
R³ peut représenter aussi avec R⁴, dans la mesure où ils sont liés à des atomes de carbone voisins, 1,3-propylène, méthylènedioxy ou difluorométhylènedioxy, et
R⁵ représente l'hydrogène et
R⁶ représente l'hydrogène, (C₁₋₈-alkyl)oxycarbonyle ou C₁₋₈-alkylcarbonyle,
leurs tautomères, leurs stéréoisomères, leurs mélanges et leurs sels.

3. Glucopyranosyloxy-pyrazoles de formule générale 1 selon la revendication 1 où
R¹ représente tétrahydrofuran-3-yle, tétrahydropyran-4-yle ou tétrahydrofuran-2-ylméthyle,
R² représente méthyle,
R³ représente méthyle, éthyle, méthoxy, éthoxy, difluorométhoxy, tétrahydrofuran-3-yloxy, tétrahydropyran-4-yloxy ou tétrahydrofuranylméthyloxy,
R⁴ représente l'hydrogène ou le fluor,
R⁵ représente I'hydrogène et
R⁶ représente l'hydrogène, méthoxycarbonyle ou éthoxycarbonyle,
leurs tautomères, leurs stéréoisomères, leurs mélanges et leurs sels.

4. Glucopyranosyloxy-pyrazoles de formule générale I selon la revendication 1 où
R¹ représente tétrahydrofuran-3-yle ou tétrahydropyran-4-yle,
R² représente méthyle,
R³ représente méthyle, éthyle, méthoxy, éthoxy ou difluorométhoxy,
R⁴ représente l'hydrogène ou le fluor,
R⁵ représente l'hydrogène,
R⁶ représente l'hydrogène, méthoxycarbonyle ou éthoxycarbonyle et
R^{7a}, R^{7b} et R^{7c} représentent l'hydrogène,
leurs tautomères, leurs stéréoisomères, leurs mélanges et leurs sels.

5. Composés de formule générale I selon la revendication 1 choisis dans le groupe consistant en
(a) 1-((S)-tétrahydrofuran-3-yl)-3-(β-D-glucopyranosyloxy)-4-(4-éthylbenzyl)-5-méthyl-1 H-pyrazole,
(b) 1-(tétrahydropyran-4-yl)-3-(β-D-glucopyranosyloxy)-4-(4-éthylbenzyl)-5-méthyl-1 H-pyrazole,
(c) 1-(tétrahydropyran-4-yl)-3-(β-D-glucopyranosyloxy)-4-(4-méthoxybenzyl)-5-méthyl-1 H-pyrazole,
ainsi que leurs dérivés où R⁶ présente une signification selon l'invention, différente de l'hydrogène, en particulier R⁶ représente éthoxycarbonyle ou méthoxycarbonyle,
y compris leurs tautomères, leurs stéréoisomères, leurs mélanges et leurs sels.

6. Sels physiologiquement acceptables des composés selon au moins l'une des revendications 1 à 5 avec des acides inorganiques ou organiques.

7. Utilisation d'un composé selon une ou plusieurs des revendications 1 à 5 ou d'un sel physiologiquement acceptable selon la revendication 6 comme médicament.

8. Utilisation d'au moins un composé selon une ou plusieurs des revendications 1 à 5 ou d'un sel physiologiquement acceptable selon la revendication 6 pour la production d'un médicament qui convient pour le traitement ou la prophylaxie des maladies ou affections qui peuvent être influencées par l'inhibition du cotransporteur de glucose dépendant du sodium SGLT.

9. Utilisation d'au moins un composé selon une ou plusieurs des revendications 1 à 5 ou d'un sel physiologiquement acceptable selon la revendication 6 pour la production d'un médicament qui convient pour le traitement ou la prophylaxie des maladies métaboliques.

10. Utilisation selon la revendication 9 **caractérisée en ce que** la maladie métabolique est choisie dans le groupe consistant en le diabète sucré de type 1 et de type 2, les complications diabétiques, l'acidose ou la cétose métabolique, l'hypoglycémie réactive, l'hyperinsulinémie, les troubles du métabolisme du glucose, la résistance à l'insuline, le syndrome métabolique, les dyslipidémies de différentes origines, l'athérosclérose et les maladies apparentées, l'obésité, l'hypertension artérielle, l'insuffisance cardiaque chronique, les oedèmes et l'hyperuricémie.

11. Utilisation d'au moins un composé selon au moins l'une des revendications 1 à 5 ou d'un sel physiologiquement acceptable selon la revendication 6 pour la production d'un médicament pour inhiber le cotransporteur de glucose dépendant du sodium SGLT.

12. Utilisation d'au moins un composé selon au moins l'une des revendications 1 à 5 ou d'un sel physiologiquement acceptable selon la revendication 6 pour la production d'un médicament pour prévenir la dégénérescence des cellules bêta pancréatiques et/ou pour améliorer et/ou rétablir la fonctionnalité des cellules bêta pancréatiques.

13. Utilisation d'au moins un composé selon au moins l'une des revendications 1 à 5 ou d'un sel physiologiquement acceptable selon la revendication 6 pour la production de diurétiques et/ou d'antihypertenseurs.

14. Procédé de production d'un médicament selon la revendication 8 **caractérisé en ce qu'**un composé selon au moins l'une des revendications 1 à 5 ou un sel physiologiquement acceptable selon la revendication 6 est incorporé dans un ou plusieurs vecteurs et/ou diluants inertes par voie non chimique.

15. Procédé de production des composés de formule générale I selon les revendications 1 à 5 **caractérisé en ce que**
a) pour la production de composés de formule générale I où R¹ représente l'un des groupes cités dans les revendications 1 à 5,
un composé de formule générale 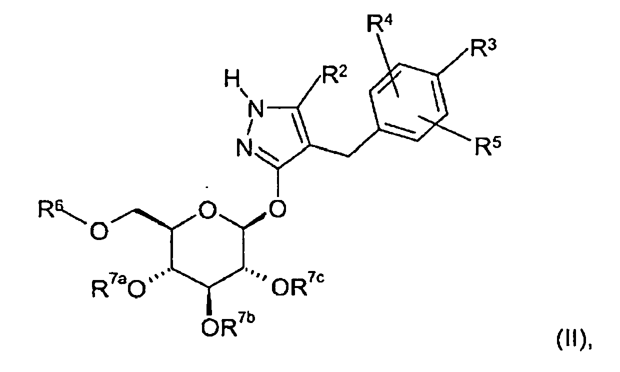 où
R² à R⁶ et R^{7a}, R^{7b}, R^{7c} sont définis comme indiqué dans les revendications 1 à 5, est mis à réagir avec un composé de formule générale
Z¹-R^{1'} (III)
où
R^{1'} possède les significations citées au début pour R¹ et Z¹ représente un groupe partant, ou
b) pour la production de composés de formule générale I où R³ représente C₁₋₆-alcoxy, C₃₋₆-cycloalkyl-oxy, C₃₋₅-cycloalkyl-C₁₋₃-alcoxy éventuellement substitué
ou a une signification choisie dans le groupe A qui est défini selon la revendication 1,
un composé de formule générale 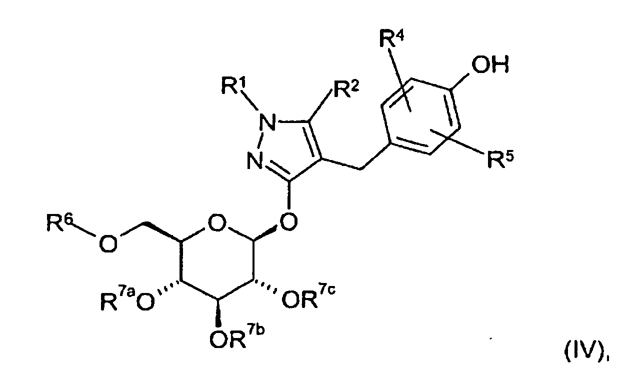 où
R¹, R² et R⁴ à R⁶ ainsi que R^{7a}, R^{7b}, R^{7c} sont définis comme dans la revendication 1, est mis à réagir avec un composé de formule générale
Z² - R^{3'} (V)
où
R^{3'} représente C₁₋₆-alcoxy, C₃₋₆-cycloalkyl-oxy, C₃₋₅-cycloalkyl-C₁₋₃-alcoxy éventuellement substitué ou a une signification choisie dans le groupe A qui est défini comme indiqué précédemment, et Z² représente un groupe partant, de préférence un atome d'halogène, par exemple un atome de chlore ou de brome, un groupe sulfonyloxy, par exemple un groupe méthanesulfonyloxy ou p-toluènesulfonyloxy ou un groupe hydroxy, et
si on le souhaite, un composé de formule générale I ainsi obtenu, où R⁶ représente un atome d'hydrogène, est converti par acylation en un composé acylé de formule générale I correspondant, et/ou
si nécessaire, un groupement protecteur utilisé dans les réactions décrites précédemment est clivé et/ou
si on le souhaite un composé de formule générale I ainsi obtenu est séparé en ses stéréoisomères et/ou
un composé de formule générale I ainsi obtenu est converti en ses sels, en particulier pour l'utilisation pharmaceutique en ses sels physiologiquement acceptables.
